# EUROPÄISCHE PATENTANMELDUNG

(11) **EP 1 517 146 A2**
(43) Veröffentlichungstag der Anmeldung: **23.03.2005**
(21) Anmeldenummer: 04104608.7
(22) Anmeldetag: 22.09.2004
(51) Int. Cl.: G01N 33/574, C12Q 1/68, A61K 39/00, A61K 48/00

(54) **Flamingo (Ant)agonisten zur Diagnose und Behandlung von Krebs**

(30) Priorität: 22.09.2003 DE 10345009
(71) Anmelder: Hinzmann, Bernd, Dr., 13127 Berlin (DE); Rosenthal, André, Prof., 14482 Potsdam (DE); Heiden, Esmeralda, 10589 Berlin (DE); Lichtner, Rosemarie, Dr., 10823 Berlin (DE)
(72) Erfinder: Hinzmann, Bernd, Dr., 13127 Berlin (DE); Rosenthal, André, Prof., 14482 Potsdam (DE); Heiden, Esmeralda, 10589 Berlin (DE); Lichtner, Rosemarie, Dr., 10823 Berlin (DE)

(57) **Zusammenfassung**

Die Erfindung betrifft Verwendungen von Flamingo zur Diagnose und Behandlung von Krebs, insbesondere des Brust-, Uterus- und/oder Ovarkarzinoms, sowie zum Screenen nach Substanzen für solche Zwecke.

## Beschreibung

### Technisches Gebiet

### Gebiet der Erfindung

Die Erfindung betrifft neue Verwendungen von Flamingo oder daraus abgeleiteten Sequenzen zum Screenen nach daran bindenden Substanzen, sowie die Verwendung von an Flamingo bindenden Substanzen zur Diagnose und/oder Behandlung von Krebs, insbesondere des Brust-, Ovar- und/oder Uteruskarzinoms.

### Stand der Technik

### Hintergrund der Erfindung und Stand der Technik

Flamingo ist beispielsweise als Seq.-ID 44 (und in seiner verlängerten Form als Seq.-ID 153) aus der Literaturstelle PCT/DE99/00908 bekannt. Für Flamingo werden folgende Synonyme ver wendet: CELSR1, Flamingo 2, FM12. Flamingo ist ein 7 Transmembran (TM) Cadherin, das vor allem an den Zell-Zell Grenzen im Drosophila Flügel beschrieben wurde und eine entscheidende Rolle bei der planaren Zell Polarität (PCP) spielt (Usui et. al, 1999, Cell 98 (5): 585-95). Im humanen Genom sind bislang drei Flamingo Homologe (CELSR1, CELSR2 und CELSR3) identifiziert worden. Das hier beschriebene Gen CELSR1 ist auf dem Chromosom 22q13 lokalisiert (Wu et al. 2000, PNAS 97 (7):3124-3129). Alle drei CELSR-Gene gehören zu einer Subfamilie der Caherin Superfamilie, die im Gegensatz zu den klassischen Cadherinen nicht mit Cateninen interagiert (Nollet *et al*. 2000, JMB 299 (3):551-572). Das besondere dieser Cadherine ist ihre 7 TM Domäne, die Homologie zu G-gekoppelten Rezeptoren aufweist. Des weiteren besitzt Flamingo neun extrazelluläre Protocadherin Domänen, welche nicht das für andere Protocadherine charakteristische Leucin in ihrer Mitte aufweisen, sondern stattdessen jeweils Isoleucin, welches auch in den Ektodomänen von N-Cadherin konserviert ist. Weiterhin besitzt Flamingo acht epidermale growth factor (EGF)-ähnliche Domänen und zwei Laminin A-G Domänen (Wu et al. 2000, PNAS 97 (7): 3124-3129). Flamingo ist mit einem offenen Leseraster (ORF von 9045 bp (SEQ-ID 1) außergewöhnlich groß (NCBI-Daten Bank: AF231024) und kodiert für ein Protein mit 3014 Aminosäuren (SEQ-ID 2).

Im Rahmen der Organisation der planaren Zellpolarität im Drosophila Flügel und Auge reguliert Flamingo unter anderem auch die Gene frizzled (fz) und dishevelled (dsh) (Ugui et al. 1999, Cell 98(5):585-95; Shimada et ;al. 2001, Curr Biol II(11):859-63; Adler et al. 2001, Curr Opin Cell Biol 13(5):635-40, Strutt et al. 2001, Curr Biol II(13):R506-9). Der Signalweg, der u.a. die Aktivierung/Inhibition von Jun-Kinase (JNK) steuert, ist einer von drei bekannten wnt Signaltransduktionswegen. Der klassische wnt/β-catenin Signalweg führt zur Aktivierung der LEF/TCF Transkriptionsfaktoren, der wnt/Ca2+ pathway führt über eine intrazelluläre Ca2+ Ausschüttumg zur Aktivierung der Proteinkinase und der wnt/JNK/PCP pathway wirkt auf das Cytoskelett (Huelsken et al. 2002,J Cell Sci. 115(Pt21):3977-8; Pandur et al. 2002, Bioessays 24(10):881-4). Der JNK/PCP Signalweg wird vermutlich über die Liganden wnt11 und wnt5a reguliert (Marlow al. 2002, Curr Biol. 12(11):876-84; Schwarz-Romond et al. 2002, Genes Dev. (16):2073-84; Yamanaka et al. 2002, EMBO Rep 3(1):69-75; Habas et al 2003, Genes Dev 17(2):295-309; Pandur et al. 2002, Nature 418(6898):636-41).

Neben JNK sind an dem PCP Signalweg ebenfalls die Rho-associated kinase (ROK) und RhoA (GTPase) beteiligt (Winter et al. 2001, Cell 105(1):81-9; Strutt et al. 2001, Curr Biol 11(13): R506-9).

Der JNK/PCP/Signalweg ist sowohl in humanen Zellinien (HEK293, Saos) als auch in Vertebraten (Zebrafisch, Xenopus) identifiziert worden (Habas et al. 2001, Cell 107(7):843-54; Yamanaka et al. 2002, EMBO Rep 3(1):69-75; Lisovsky et al. 2002, Curr Biol 12(1):53-8; Wiggan et al. 2002, J Cell Sci 115(Pt 3):531-41; Marlow et al. 2002, Curr Biol 12(11 ):876-84; Schwarz-Romond et al. 2002, Genes Dev. 16(16):2073-84; Takeuchi et al. 2003, Curr Biol. 13(8):674-9; Habas et al. 2003, Genes Dev 17(2):295-309).

Die Aktivierung des JNK/PCP Signalweges durch das Xenopus frizzied8-Gen (Xfz8) führt bei Xenopus Embryos zur Apoptose (Lisovsky et al, 2002, Curr Biol 12(1):53-8). Erst kürzlich wurde diskutiert, dass Flamingo auch den klassischen wnt/β-catenin pathway beeinflussen kann (Morgan et al. 2003, Int J Dev Biol. 47(4):245-52).

In Drosophila Ommatidien ist Flamingo in einem anderen Signalweg involviert. Über eine Interaktion mit dem Notch-Rezeptor kommt es zu einem Ungleichgewicht des frizzled (fz)-Signals in R3 und R4 Zellen (Strutt et al. 2002, Curr Biol 12(10):813-24; Das et al. 2002, Dev Cell 2 (5):655-66).

Des Weiteren konnte gezeigt werden, dass Flamingo einen Einfluß auf die neuronale Morphogenese in Drosophila besitzt. Flamingo beeinflusst den Axon-Abstand sowie die korrekte Verbindung von Axon und Targetzelle. Eine Überexpression von Flamingo führt zum Zurückziehen der Axone von den Targetzellen (Senti et al. 2003, Curr Biol 13(10):828-32; Reuter et al. 2003, Development 130(6):1203-13; Lee et al. 2003, Nat Neurosci 6(6) 557-63).

Obwohl die Expression von Flamingo in Vertebraten beschrieben wurde, ist über seine Funktion wenig bekannt (Morgan et al. 2003, Int J Dev Biol. 47(4):245-52). Es wird spekuliert, dass es eine Rolle während der Gastrulation und im sich bildenden Nervensystem spielt (Hadjantonakis et al. 1997, Genomics 45(1):97-104; Shima et al. 2002, Dev Dyn 223(3):321-32; Tissir et al. 2002, Mech Dev 112(1-2):157-60; Formstone et al. 2001, Mech Dev. 109(1)-.91-4).

Krebs ist eine meist letal verlaufende Erkrankung mit zunehmender Inzidenz. Daher ist es wünschenwert, verbesserte Ansätze zur Diagnose und Therapie von Krebserkrankungen zur Verfügung zu stellen.

### Ausführungsform(en) der Erfindung

### Technisches Problem der Erfindung

Der Erfindung liegt daher das technische Problem zugrunde, pharmazeutische Zusammensetzungen zur Diagnose und/oder zur Behandlung von Krebs, insbesondere von Brust-, Ovar- und/oder Uteruskarzinomen, anzugeben, sowie Mittel zu deren Identifizierung.

Grundzüge der Erfindung und bevorzugte Ausführungsformen.

Zur Lösung dieses technischen Problems lehrt die Verwendung einer für Flamingo codierenden Nukleinsäure und/oder eines Flamingo Peptids oder Proteins zur Detektion von Krebs, insbesondere von Brust-, Ovar-, und/oder Uteruskarzinomen oder zur Detektion eines Risikos der Erkrankung an einem solchen Karzinom oder zur Detektion eines Risikos einer Progression eines solchen Karzinoms, wobei eine Gewebeprobe, insbesondere eine Brust-, Ovar- und/oder Uterus-Gewebeprobe, auf Transkription oder Übertranskription von Flamingo RNA oder auf Expression oder Überexpression eines Flamingo Proteins untersucht wird.

Eine an für Flamingo codierende Nukleinsäure oder eine an Flamingo Protein oder Peptid bindende Detektorsubstanz, vorzugsweise enthaltend eine Reportergruppe, kann verwendet werden, wobei Bindung besagter Nukleinsäure und/oder besagten Proteins oder Peptids an die Detektorsubstanz halbquantitativ oder quantitativ detektiert wird. In diesem Zusammenhang lehrt die Erfindung weiterhin ein Testsystem zur (in vitro) Detektion eines vorstehend genannten Karzinoms oder eines Risikos der Erkrankung hieran oder der Progressionsprognose, enthaltend Mittel zur quantitativen Messung der Expression von Flamingo in Gewebeproben, wobei diese Mittel beispielsweise Mittel zur Amplifikation und spezifischen Detektion von Flamingo RNA und/oder eine Detektorsubstanz, insbesondere spezifisch für Flamingo Protein, sein können.

Die Erfindung lehrt weiterhin die Verwendung einer Flamingo RNA oder eines Flamingo Proteins oder Peptids zum Screenen nach daran bindenden Substanzen, insbesondere prospektiven Wirkstoffen zur Modulierung, insbesondere Inhibierung, von besagter RNA oder besagtem Protein oder Peptid, oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird. In diesen Zusammenhängen lehrt die Erfindung weiterhin ein Screeningsystem zur Ermittlung von für die Behandlung von vorstehenden Tumorerkrankungen geeigneten Wirksubstanzen enthaltend eine Flamingo Nukleinsäure oder ein Flamingo Protein bzw. Peptid, Mittel zur Bestimmung von (in vitro) Bindungsereignissen an die Flamingo Nukleinsäure oder an das Flamingo Protein bzw. Peptid, und/oder Mittel zur Bestimmung der (in vitro) Aktivität von Flamingo Protein. Hierbei kann Flamingo in einem zellfreien oder einem zellbasierten System, letzteres insbesondere aufweisend Zellen aus vorstehend genannten Geweben bzw. eine hieraus entwickelte Zelllinie, vorliegen. Mittel zur Bestimmung von Bindungsereignissen können beispielsweise natürlicherweise in normalen oder in Tumorzellen z.B. an Flamingo Protein bindende Substanzen bzw. Assoziationspartner umfassen, wobei über deren (freie) Konzentration bzw. Konzentrationsänderung bei Zugabe prospektiver Wirksubstanzen und/oder Detektorsubstanzen eine kompetitive Bindung einer bindenden Wirk- oder Detektorsubstanz bestimmt wird. Solche Mittel können aber auch physikalische bzw. physikalisch-chemische Methoden umfassen, wie beispielsweise Röntgenstrukturanalyse und/oder NMR, insbesondere zweidimensionale 1H/1H oder 15N/1H oder 14C/1H Korrelationsspektroskopie. Hierbei werden Spektren vor und nach der Zugabe einer prospektiven Wirk- oder Detektorsubstanz miteinander verglichen und im Falle von Änderungen ist ein Bindungsereignis festgestellt. Es kann mit Spektren oder dergleichen entweder von Flamingo oder der prospektiven Substanz oder mit einer Kombination aus beidem gearbeitet werden. Selbstverständlich sind auch alle anderen fachüblichen Methoden der Bestimmung von Bindungsereignissen und/oder Proteinaktivitäten einsetzbar. Beispielsweise kann eine prospektive Substanz (oder mehrere Substanzen, räumlich voneinander getrennt) immobilisiert sein, wobei dann markiertes Flamingo aufgetragen wird. Ein Bindungsereignis wird dann nach Auftrag und folgender Spülung durch Detektion, ggf. ortlich aufgelöst, der Markierung gebundenen Flamingos festgestellt. Bindungsereignisse können auch ohne Markierung eines der Bindungspartner mittels der dem Fachmann geläufigen Biacore Technologie (Oberflächenplasmonen Resonanz)detektiert werden. Umgekehrt kann Flamingo immobilisiert sein und es wird eine markierte prospektive Substanz oder eine Mischung hieraus aufgetragen. Bindungsereignisse werden analog der vorstehenden Variante festgestellt.

Die Erfindung lehrt schließlich die Verwendung einer Flamingo inhibierenden oder daran bindenden Substanz zur Herstellung einer pharmazeutischen Zusammensetzung zur Behandlung und/oder Diagnose von Krebs, insbesondere Brust-, Ovar-, Und/oder Uteruskarzinomen.

Die Substanz kann ein Antikörper sein, welcher durch Immunisierung eines nicht-menschlichen Säugetiers mit einem Flamingo Peptid oder Protein, mit hierfür codierender cDNA transfizierte Zellen, cDNA Immunisierung (Genovac), mit endogen ein solches Peptid oder Protein exprimierenden Tumorzellen, oder mit rekombinant hergestellten Flamingo Peptiden oder Proteinen, erhältlich ist, oder ein Phage-Display-Antikörper sein. Die Substanz kann aber auch eine Mimikryverbindung eines Antikörpers gegen ein Flamingo Peptid oder Protein sein. Die Substanz kann schließlich ein Aptamer, eine antisense RNA, ein Ribozym oder eine siRNA gegen Flamingo Nukleinsäuren sein. Die Substanz kann zusätzlich eine zytotoxische und/oder immunstimulierende Komponente tragen.

Bevorzugt ist es, wenn die vorstehenden, an Flamingo Protein bindenden Substanzen in der Verwendung zu therapeutischen Zwecken spezifisch an das Flamingo Protein binden und es in seiner biologischen Aktivität modulieren. Dies ist nicht erforderlich im Falle der Fusion bzw. Verbindung der Substanz mit einer zytotoxischen Komponente. Dies ist weiterhin nicht erforderlich, wenn die Substanz der Gewinnung eines anti-idiotypischen Antikörpers dient, welcher vom Immunsystem eines Patienten aufgrund seiner nicht-humanisierten Form als körperfremd erkannt wird und dem Immunsystem ansonsten ein Flamingo-Antigen präsentiert.

Die pharmazeutische Zusammensetzung kann zur beliebigen Applikation, beispielsweise i.v. oder i.p. Injektion, hergerichtet sein. Eine Herrichtung zur lokalen Applikation in Tumorzellen enthaltendem Gewebe wird sich empfehlen im Falle des Einsatzes einer zytotoxischen Komponente.

Die Erfindung läßt sich im Rahmen eines Verfahrens zur Diagnose bzw. (Progressions-) Prognose einer Tumorerkrankung verwenden, wobei eine Detektorsubstanz in einer Ausführungsform mit einer Reportergruppe in zu untersuchendes Gewebe, ggf. in vitro nach Gewebeentnahme, appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert bzw. als progressionsgefährdet oder nicht progressionsgefährdet eingestuft wird, sowie eines Verfahrens zur Behandlung einer Krebserkrankung, wobei eine erfindungsgemäße pharmazeutische Zusammensetzung in einer physiologisch wirksamen Dosis einem Patienten dargereicht wird. Im Falle der Diagnose bzw. Progressionsprognose kann zusätzlich oder alternativ eine Gewebeprobe mit einem erfindungsgemäßen Testsystem auf Flamingo Expression untersucht werden.

Die Erfindung beruht insbesondere auf der Erkenntnis, daß Flamingo in Brust-, Ovar- und/oder Uterusgeweben unterschiedlich exprimiert wird, i.e. in besagten Tumorgeweben ist die Expression im Falle solcher Karzinome höher, verglichen mit normalen Zellen gleichen Gewebes und der daraus herleitbaren technische Lehre, daß Flamingo als Zielmolekül bei der Diagnostik und Therapie bzw. Prophylaxe besagter Tumorerkrankungen eingesetzt werden kann. Flamingo kann also als spezifischer Marker zur Identifizierung von Tumorzellen in den besagten Tumorgeweben dienen. Es ist erkannt worden, dass pathologisch normale Zellen mit einer erhöhten Expression ein erhöhtes Risiko aufweisen, zu Krebszellen zu transformieren. Auf der anderen Seite bietet die Inhibierung von Flamingo die Möglichkeit, in die Tumor-spezifischen Flamingo Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Tumorzellen, insbsondere aber einer Hemmung der Transformation zu Tumorzellen, beizutragen.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von Flamingo zu untersuchen. Alternativ kann mit einer erfindungsgemäßen Detektorsubstanz zur Diagnose in vivo auf Flamingo Abhängigkeit getestet werden. Wird eine Expression bzw. Überexpression von Flamingo gegenüber Normalgewebe gleichen Typs festgestellt, so ist die Anwendung der erfindungsgemäßen pharmazeutischen Zusammensetzung indiziert.

Generell ist es im Rahmen der Erfindung möglich, patientenspezifisch auf differentielle Expression zu untersuchen, wobei Normalgewebeprobe und Tumorgewebeproben bzw. tumorverdächtige Gewebeproben dem (gleichen) Patienten entnommen und vergleichend auf Werte der Flamingo Expression untersucht werden.

Handelt es sich bei dem Tumor um einem Typus, bei welchem Tumorzellen Flamingo exprimieren, Normalzellen gleichen Gewebetyps jedoch nicht, so ist es besonders bevorzugt, wenn die an Flamingo bindende Substanz zusätzlich eine zytotoxische und/oder immunstimulierende Komponente trägt. Dies führt dann letztendlich dazu, dass praktisch ausschließlich Tumorzellen getötet werden, sei es durch die Zytotoxizität, sei es durch Angriff durch das stimulierte Immunsystem, während Normalzellen in dem Gewebe praktisch vollständig erhalten bleiben. In dieser Ausführungsform braucht die bindende Substanz selbst nicht inhibierend auf Flamingo zu wirken, da die bindende Substanz dann lediglich als Marker funktionieren muß, welcher die Komponenten zu Ziel-Tumorzellen trägt. Im Falle des Einsatzes einer zytotoxischen Komponente wird es sich besonders empfehlen, wenn die pharmazeutische Zusammensetzung zur lokalen Applikation in Tumorzellen enthaltendem Gewebe hergerichtet ist, beispielsweise zur Injektion.

Die Erfindung lehrt schließlich ein Protein oder Peptid enthaltend die Sequenz SEQ-ID 37, nämlich

SEQ-ID 37: GSNETSI,

oder eine Nukleinsäure codierend für ein solches Peptid.

Dabei kann es sich beispielsweise um die Sequenz SEQ.-ID 4 oder die Sequenz SEQ-ID 3 handeln. Diese erfindungsgemäße Flamingo Spleiß-Variante hat einen ORF von 9057 bp (SEQ-ID 3) und kodiert ein Protein von 3019 AS (SEQ-ID 4). Diese Variante ist am 3'-Ende gegenüber AF231024 verlängert.

Definitionen und weitere Ausführungsformen der Erfindung.

Flamingo Sequenzen, insbesondere Teilsequenzen, welche in im Rahmen der Erfindung nutzbare Nukleinsäuren oder Peptide oder Proteine enthalten sind, sind in den Seq.-ID 1 bis 36 dargestellt. Die im Rahmen der Erfindung nutzbaren Nukleinsäuren oder Peptide oder Proteine können auch aus diesen Sequenzen bestehen. Flamingo Sequenzen sind im Rahmen der Erfindung auch an Flamingo Nukleinsäuren bindende bzw. diese inhibierende Nukleinsäuresequenzen.

Im Rahmen dieser Beschreibung wird die Bezeichnung Flamingo für alle humanen Isoformen, bekannt oder neu, auf Nukleinsäuren- oder Aminosäurenbasis, verwendet. Mit diesen Begriffen mit umfaßt sind auch die im Rahmen dieser Beschreibung offenbarten kurzen Sequenzen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt, berechnet mit dem Programm MEGALIGN (DNASTAR LASERGENE) in der zum Zeitpunkt der vorliegenden Anmeldung aktuellen Fassung. Im Falle der Nukleinsäuresequenzen sind auch komplementäre oder allelische Varianten mit umfaßt. Weiterhin sind Sequenzen umfaßt, welche lediglich Teilsequenzen der explizit offenbarten Sequenzen, beispielsweise ein Exon oder mehrere Exons, oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 Basen, aufweisen und im Falle der Proteine bzw. Peptide mit zumindest gleicher Affinität an ein protein- oder peptidspezifisches Zielmolekül binden. Weiterhin sind alle mit erfindungsgemäßen Nukleinsäuren hybridisierende Nukleinsäuren umfaßt, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer operativ verbundenen Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Flamingo Nukleinsäuren oder Protein bzw. Peptide neben den Volllängen der offenbarten Sequenzen (siehe auch vorstehender Absatz) auch Teilsequenzen hieraus, und zwar mit einer Mindestlänge von 12 bis 30 Nukleotiden, vorzugsweise 30 bis 90 Nukleotiden, im Falle der Nukleinsäuren und einer Mindestlänge von 4 bis 10 Aminosäuren, vorzugsweise 10 bis 30 Aminosäuren, im Falle der Peptide oder Proteine. Diese Teilsequenzen können in ansonsten von Flamingo verschiedene Nukleinsäuren- oder Protein- bzw. Peptidsequenzen eingebaut sein.

Der Begriff der Behandlung umfaßt auch die Prophylaxe, insbesondere die Prophylaxe der Progression zu Tumoren.

Als Inhibitor ist eine Verbindung oder Substanz bezeichnet, welche entweder die Bildung von Flamingo Protein inhibiert oder gebildetes Flamingo Protein in der Aktivität reduziert, bezogen auf die Flamingo Aktivität in Abwesenheit des Inhibitors. Insofern kann ein Inhibitor einerseits eine Substanz sein, welche in der Entstehungskaskade von Flamingo inhibierend eingreift. Auf der anderen Seite kann ein Inhibitor eine Substanz sein, welche mit gebildetem Flamingo eine Bindung eingeht, und zwar dergestalt, dass weitere physiologische Wechselwirkungen mit endogenen Substanzen zumindest reduziert sind.

Mimikry-Moleküle sind Verbindungen, die den variablen Bereich, insbesondere den Bindungsbereich eines Antikörpers, nachbilden und an gleicher Stelle eines Zielmoleküls binden, wie der zu Grunde liegende Antikörper.

Der Begriff der Antikörper umfaßt polyklonale Antikörper, monoklonale Antikörper, nicht-humane, humane und humanisierte Antikörper, sowie Phage-Display-Antikörper, aber auch chimäre Antikörper sowie spezifische Fragmente der leichten und/oder der schweren Kette des variablen Bereiches zu Grunde liegender Antikörper vorstehender Art sowie anti-idiotypische Antikörper. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt der Begriff der Antikörper bispezifische Antikörper. Bispezifische Antikörper kombinieren eine definierte Immunzellaktivität mit einer spezifischen Tumorzellerkennung, wodurch Tumorzellen getötet werden. Ein bispezifischer Antikörper bindet einerseits an ein Auslösemolekül der Immun-Effektorzelle (z.B. CD3, CD16, CD64) und andererseits an Antigene der Tumorzielzelle.

Die galenische Herrichtung einer erfindungsgemäßen pharmazeutischen Zusammensetzung kann in fachüblicher Weise erfolgen. Als Gegenionen für ionische Verbindungen kommen beispielsweise Na+, K+, Li+ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder injizierbare Lösungen (i.v., i.p., i.m.) sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxyd, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren Flamingo differenziell, wenn Normalzellen des gleichen Gewebetyps (des gleichen oder verschiedener Probanden) dieses nicht exprimieren. Tumorzellen überexprimieren Flamingo spezifisch bzw. differenziell, wenn Flamingo im Vergleich zu Normalzellen des gleichen Gewebetyps zumindest in doppelter Menge exprimiert wird.

Zytotoxische Komponenten bzw. Gruppen sind Verbindungen, welche direkt oder indirekt Apoptose einleiten bzw. zu Nekrose führen oder zumindest wachstumshemmend wirken. Solche Gruppen bzw. Verbindungen können neben Radioisotopen (z.B. 188Re, 213Bi, 99mTc, 90Y, 131J, 177Lu) insbesondere Zytostatika sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Voncristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierenrindensteroide, Aminogluthetimid, Gestagene, Östrogene, Androgene, Antiöstrogene, Tamoxifen, Steriodanaloga, Flutamid). Bei Bindung einer solchen Verbindung mit einer an Flamingo bindenden Substanz erfolgt die Kopplung dergestalt, daß die Affinität zu Flamingo um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, -beta, -gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an Flamingo bindende Substanz kann eine Substanz sein, welche an ein Flamingo Protein oder eine Flamingo RNA bindet.

Im Rahmen der vorstehenden Definition gegenüber dem engen Wortsinn erweiterte Begriffsbestimmungen umfassen auch die bestimmten Begriffe im engen Wortsinn.

### Beispiele.

Im Folgenden wird die Erfindung anhand von lediglich bevorzugte Ausführungsformen darstellenden Beispielen und Figuren näher erläutert.

### Beispiel 1: In-situ-Hybrisierungen zur differentiellen Expression

In-situ-Hybridisierungen zeigen, dass die Überexpression des flamingo Gens in Tumoren der Brust, des Ovars und des Uterus festzustellen ist. Die Analysen erfolgten durch In-situ-Hybridisierung in den verschiedenen Geweben mit einer flamingo spezifischen Sonde (SEQ-ID 15) (Figur 1, Figur 2 (a+b), Figur 3). In 84% der Brusttumorgewebeschnitte wird eine hohe Flamingo Genexpression gemessen, während 70% der Normalgewebe eindeutig negativ sind (Tab. 1). In keinem der Normalgewebe des Ovars ist eine flamingo Expression zu messen, während in 72% der Tumorproben eine deutliche Expression nachgewiesen werden kann. In 94% der Normalgewebe des Uterus ist keine Expression nachweisbar, während hingegen 32% der Tumorgewebe als positiv gemessen wurden (Tab. 2).

Tabelle 1: Ergebnisse der RNA in-situ-Hybridisierung mit einer flamingo spezif. antisense Sonde SEQ-ID 15 auf Brusttumor- (T) und Normalgewebe (N).

Gewebe Anzahl Negativ Positiv Positiv Positiv

- + ++ +++

Brust N 30 21 (70%) 5 (17%) 2 (14%) 2 (7%)

Brust T 43 7 (16%) 14 (32%) 11 (26%) 11 (26%)

Tab. 2: Ergebnisse der RNA in-situ-Hybridisierung mit einer flamingo spezifischen antisense Sonde SEQ-ID 15 auf den Biocat Arrays (Uterusund Ovargewebe, bei Ovar wurden auch andere Gewebe verwendet).

Gewebe Anzahl Negativ Positiv Positiv Positiv

- + ++ +++

Ovar N 3 3 (100%)

Ovar T 18 5 (28%) 5 (28%) 6 (33%) 2 (11%)

Uterus N 17 16 (94%) 0 (0%) 0 (0%) 1 (6%,Pr.)

Uterus T 22 15 (68%) 5 (23%) 2 (9%) 0 (0%)

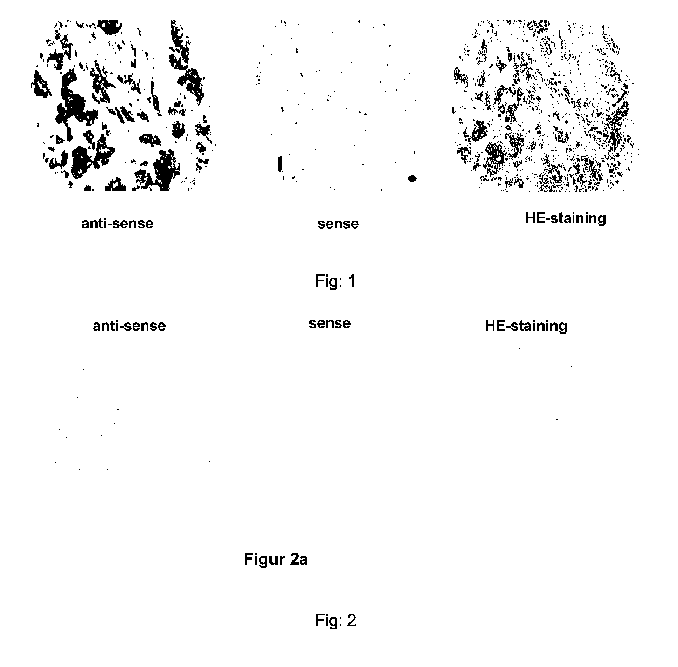

In der Figur 1 erkennt man im einzelnen eine RNA in-situ Hybridisierung zur Analyse der Überexpression von Flamingo in Brusttumorgewebe. Gewebeproben aus Brusttumorgewebe wurden mit einer Flamingo spezifischen antisense Sonde inkubiert und mit BM-Purple gefärbt. Die Gegenfärbung erfolgte mit KernechtRot. Es sind maligne und nichtmaligne Epithelzellen voneinander zu unterscheiden, von denen die malignen Zellen eine starke Anfärbung der RNA in der in situ Hybridisierung zeigen. Man erkennt in der Figur 1, dass Flamingo im Brusttumorgewebe (links) auf RNA-Ebene deutlich überexprimiert ist, verglichen zur Kontrolle (Mitte).

Figur 2a zeigt eine in-situ-Hybridisierung zur Analyse der Überexpression von Flamingo in Cervix-Tumorgewebe. Gewebeproben aus Cervixtumorgewebe wurden mit einer Flamingo spezifischen antisense Sonde inkubiert und mit BM-Purple gefärbt. Die Gegenfärbung erfolgte mit Kernecht-Rot. Es sind maligne und nichtmaligne Epithelzellen voneinander zu unterscheiden, von denen die malignen Zellen eine starke Anfärbung der RNA in der in situ Hybridi- sierung zeigen. Man erkennt in der Figur 2a, dass Flamingo im Cervixtumorgewebe (links) auf RNA-Ebene deutlich überexprimiert ist, verglichen zur Kontrolle (Mitte).

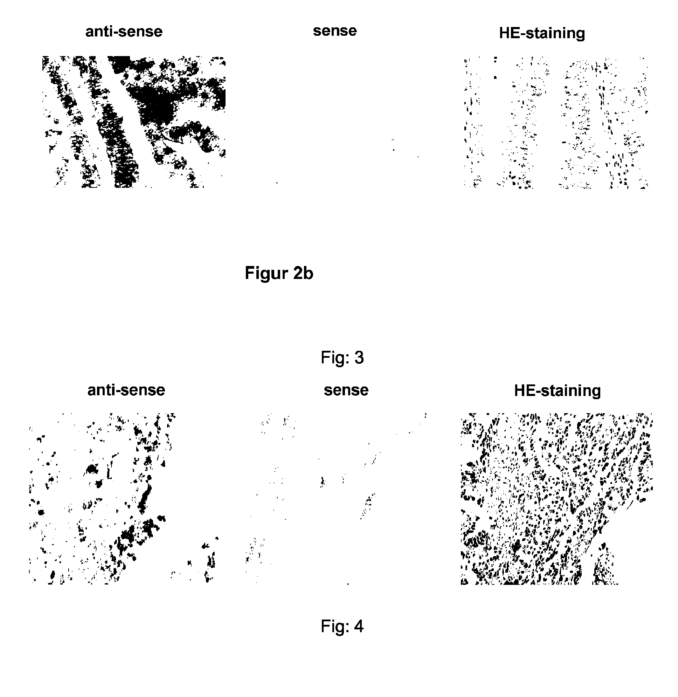

Figur 2b zeigt eine in-situ-Hybridisierung zur Analyse der Überexpression von Flamingo in Endometrium-Tumorgewebe. Gewebeproben aus Endometriumtumorgewebe wurden mit einer Flamingo spezifischen antisense Sonde inkubiert und mit BM-Purple gefärbt. Die Gegenfärbung erfolgte mit KernechtRot. Es sind maligne und nichtmaligne Epitheizellen voneinander zu unterscheiden, von denen die malignen Zellen eine starke Anfärbung der RNA in der in situ Hybridisierung zeigen. Man erkennt in der Figur 2b, dass Flamingo im Endometriumtumorgewebe (links) auf RNA-Ebene deutlich überexprimiert ist, verglichen zur Kontrolle (Mitte).

Figur 3 zeigt eine in-situ-Hybridisierung zur Analyse der Überexpression von Flamingo in Ovar Tumorgewebe. Gewebeproben aus Ovartumorgewebe wurden mit einer Flamingo spezifischen antisense Sonde inkubiert und mit BM-Purple gefärbt. Die Gegenfärbung erfolgte mit KernechtRot. Es sind maligne und nichtmaligne Epithelzellen voneinander zu unterscheiden, von denen die malignen Zellen eine starke Anfärbung der RNA in der in-situ Hybridisierung zeigen. Man erkennt in der Figur 3, dass Flamingo im Ovartumorgewebe (links) auf RNA-Fbene deutlich überexprimiert ist, verglichen zur Kontrolle (Mitte).

### Beispiel 2: Antisense und RNAi

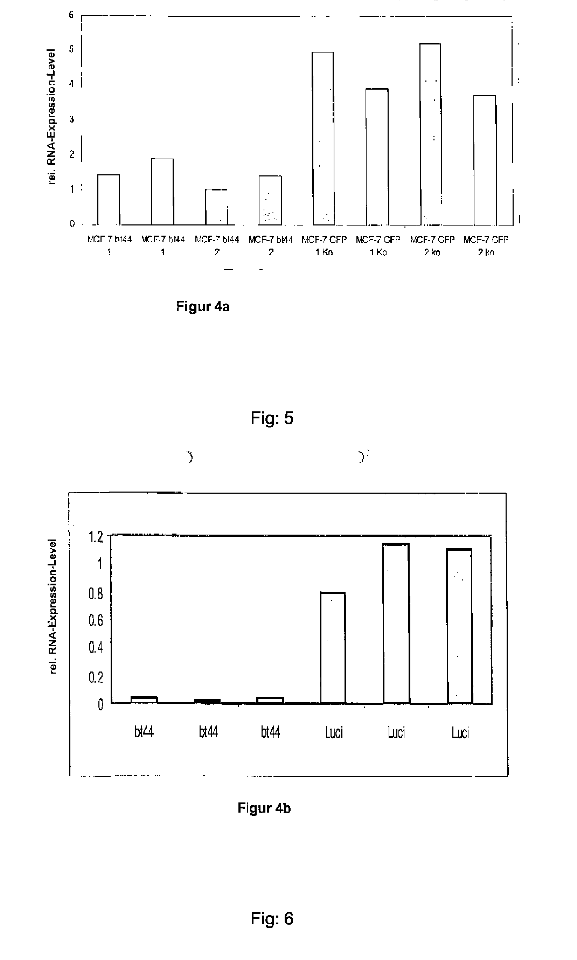

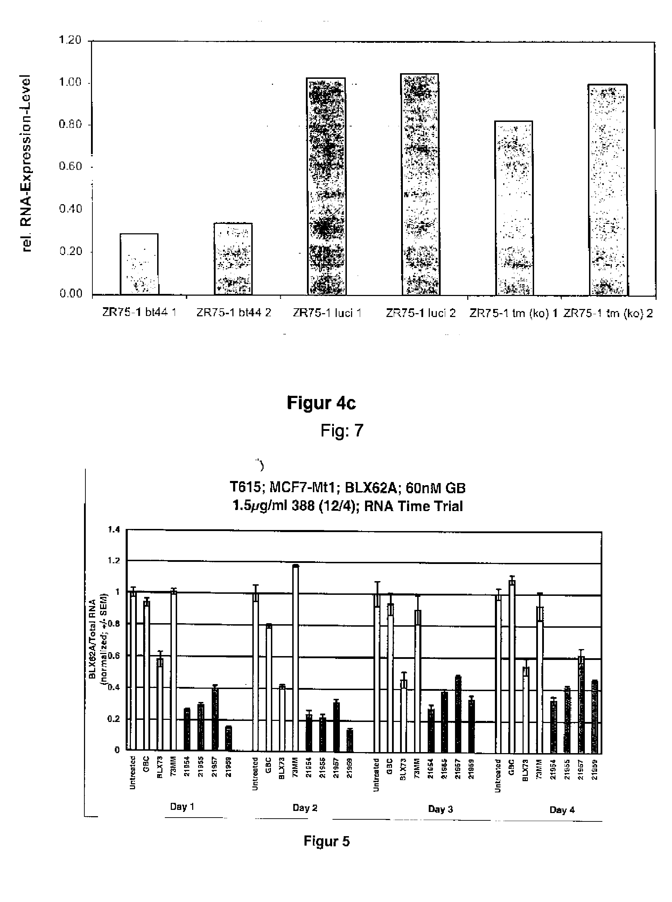

RNAi Oligos wurden von der Firma RNAx aus der Flamingo Sequenz abgeleitet und von der Firma Qiagen hergestellt. Die Transfektion der humanen Krebszelllinien mit den RNAi Oligos erfolgte mit dem Reagenz TransMessenger (Qiagen). 48 h nach der Transfektion wurde der endogene RNA-Level von Flamingo durch RT-PCR (TaqMan) mit dem QuantiTect SYBR Green RT-PCR Kit von Qiagen gemäß der Anleitung des Herstellers bestimmt. Im Rahmen der Erfindung wurde gefunden, dass die von der Nukleinsäuresequenz (SEQ-ID 1 bzw. SEQ-ID 3) abgeleitete siRNA (SEQ-ID 16) einen Flamingo-RNA-Knock-down in MCF-7 Zellen (Figur 4 (a+b)) und auch anderen Zelllinien (z.B. ZR75-1 (Figur 4c); Ovarzelllinien) bewirkt. Diese Sequenz SEQ-ID 16 kann daher therapeutisch eingesetzt werden.

Die Ergebnisse der Figur 4 wurden im Einzelnen wie folgt erhalten. Figur 4a zeigt Flamingo-Knock-down in MCF-7 Zellen durch RNAi. Dargestellt sind die Ergebnisse der Transfektionen der Flamingo-siRNAs bzw. Kontroll-GFP(green fluorescent protein)-siRNAs in MCF-7 Zellen. Es handelt sich um jeweils zwei parallel durchgeführte Transfektionen (1 und 2). Von jedem Transfektionsansatz wurden Doppelwerte des endogenen Flamingo RNA Levels mit der Real-Time-PCR-Methode (TaqMan) bestimmt. Im Ergebnis ist eine deutliche Reduktion um den Faktor 2,5 bis 5 der Flamingo RNA in den mit den Flamingo spezifisch behandelten RNAis zu messen. Figur 4b zeigt flamingo-Knock-down in MCF-7 Zellen durch RNAi. Dargestellt sind die Ergebnisse der Transfektionen der Flamingo-siRNAs bzw. Kontroll-Luciferase-siRNAs in MCF7-Zellen. Es wurden jeweils drei Transfektionen parallel durchgeführt. Von jeder Transfektion wurden Dreifachwerte des endogenen Flamingo RNA Levels mit der Real-Time-PCR-Methode (TaqMan) bestimmt und hier als Mittelwert zusammengefasst. Im Ergebnis ist eine deutliche Reduktion der Flamingo RNA um den Faktor 16 bis 22 in den mit den Flamingo spezifisch behandelten RNAis zu messen. Figur 4c zeigt Flamingo-Knock-down in ZR75-1 Zellen durch RNAI. Dargestellt sind die Ergebnisse der Transfektionen der Flamingo-siRNAs bzw. Kontroll-Luciferase-siRNAs sowie Kontrollen mit nur Transmessenger-Reagenz in ZR75-1 Zellen. Es handelt sich um jeweils zwei parallel durchgeführte Transfektionen (I und 2). Von jedem Transfektionsansatz wurden Doppelwerte des endogenen Flamingo RNA Levels mit der Real-Time-PCR-Methode (TaqMan) bestimmt. Im Ergebnis ist eine deutliche Reduktion um den Faktor 2,9 bis 3,4 der Flamingo RNA in den mit den Flamingo spezifisch behandelten RNAis zu messen.

Es wurden weiterhin stabile Zelllinien mit der Sequenz SEQ-ID 16 erzeugt, indem die Sequenz in den Transkriptionsvektor pRNA-U6.1/Neo kloniert wurde (GeneScript). Als Rezipient wurden Flamingo endogen exprimierende Brust- (z.B. MCF-7, ZR75-1 oder T47-D) und Ovar-Zeilinien (T0V112 D, OV 90, PA 1, TOV 21 G) verwendet. Die stabil transfizierten Zelllinien, in denen die endogen exprimierte Flamingo RNA durch stabiles Einführen der siRNA permanent herunterreguliert wird, wurden in zellbasierten Assays und in Mausmodellen verwendet. Die Flamingo siRNA (SEQ-ID 16) kann als Oligo oder als Vektor für den gentherapeutischen Ansatz eingesetzt werden.

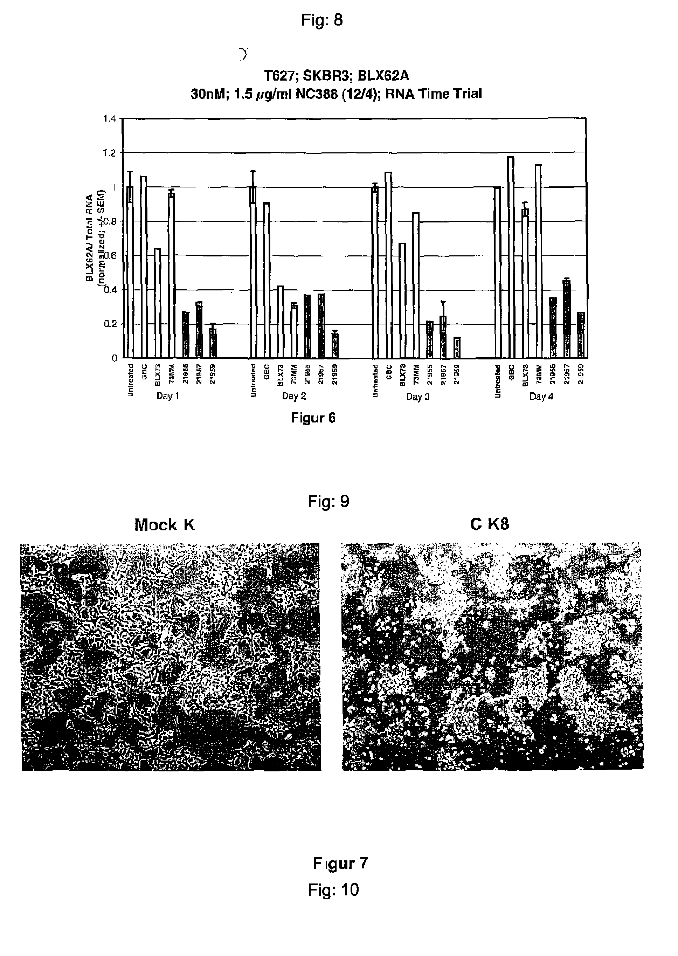

Weiterhin wurden verschiedene Antisense Moleküle (RPI/Atugen, Boulder, USA) (GeneBlocs) sowie deren Kontrolloligos (Missmatch-Oligos) in zellbasierten Assays auf ihre hemmende Wirkung auf die endogene Flamingo RNA getestet (SEQ-ID 17, SEQ-ID 18, SEQ-ID 19, SFQ-ID 20, SEQ-ID 21, SEQ-ID 22, SEQ-ID 23, SEQ-ID 24, SEQ-ID 25, SFQ-ID 26, SEQ-ID 27, SEQ-ID 28, SEQ-ID 29, SEQ-ID 30, SEQ-ID 31, SEQ-ID 32, SEQ-ID 33, SFQ-ID 34). Die erfindungsgemäßen Sequenzen SEQ-ID 17, SEQ-ID 19, SEQ-ID 21, SEQ-ID 25 zeigen einen RNA-knock-down in MCF-7- und SKBR3-Zellen (Figur 5 und Figur 6). Auch diese Sequenzen wurden für zelluläre Assays und Mausmodelle verwendet. Die Flamingo Teilsequenzen SEQ-ID 17, SEQ-ID 19, SEQ-ID 21, SEQ-ID 25 sind als Oligo oder kloniert in einen Vektor für den gentherapeutischen Ansatz geeignet.

Figur 5 zeigt Antisense-Knock-down in MCF-7. MCF-7 Zellen wurden über einen Zeitraum von vier Tagen mit 60 nM antisense Oligos behandelt und anschließend auf ihre endogene Expression des Flamingo Gens (hier als BLX62A bezeichnet) analysiert. Im Ergebnis ist in der Figur 5 im Vergleich zu unbehandelten Zellen ("untreated") eine deutliche Abnahme der RNA um den Faktor 2-5 in den Zellen zu finden, die mit den Flamingo spezifischen Antisense Oligos (SEQ-ID 17, SEQ-ID 19, SEQ-ID 21, SEQ-ID 25) behandelt wurden. Das Oligo #21959 (SEQ-ID 21) zeigt dabei in den ersten zwei Behandlungstagen seinen stärksten Effekt. Das Kontroll-Oligo 73MM (missmatch), verhält sich in der Regel wie die unbehandelte Kontrolle. GBC ist die Verwendung des Transfektionsreagenz ohne Antisense Oligo und dient als Negativkontrolle.

Figur 6 zeigt Antisense-Knock-down in SKBR3. SKBR3 Zellen wurden über einen Zeitraum von vier Tagen mit 30 nM Antisense Oligos behandelt und anschließend auf ihre endogene Expression des flamingo Gens (hier als BLX62A bezeichnet) analysiert. Im Ergebnis ist in der Figur 6 im Vergleich zu unbehandelten Zellen ("untreated") eine deutliche Abnahme der RNA um den Faktor 2-10 in den Zellen zu finden, die mit den Flamingo spezifischen antisense Oligos (SEQ-ID 19, SEQ- ID 21, SEQ-ID 25) behandelt wurden. Das Oligo #21959 (SEQ-ID 21) zeigt dabei in den ersten drei Behandlungstagen seinen stärksten Effekt. Das Kontroll-Oligo 73MM (mismatch), verhält sich in der Regel wie die unbehandelte Kontrolle. GBC ist die Verwendung des Transfektionsreagenz ohne Antisense Oligo und dient als Negativkontrolle

### Beispiel 3: Phänotypveränderung

Der C-terminale (C) Bereich von Flamingo (SEQ-ID 35) wurde in den Vektor pSecTag2 (Invitrogen) kloniert. Dieser Vektor enthält eine Ig *K* Kette als sekretorische Leader Sequenz. In diesen kommerziellen Vektor ist ein V5-Tag eingefügt worden, so dass die inserierte flamingo-Sequenz über eine N-terminale extrazelluläre V5-Sequenz detektierbar wird. Der Kontrollvektor (Vektor ohne Flamingo-Insertion) und der Flamingo-Vektor wurden stabil in HEK293 transfiziert und auf Zeocin selektioniert. Die stabilen Mock (Zellklone mit Kontrollvektor) und C-Klone (C-terminale Domäne von Flamingo) wurden mit dem V5-Antikörper (invitrogen) im FACS und im Westernblot analysiert. 90% aller C-Klone waren stabil mit Flamingo transfiziert. Die stabilen Mock- und C-Klone zeigen einen unterschiedlichen Phänotyp (Figur 7). Während die Mock-Klone, ähnlich wie der Wildtyp, an die Platte adhärieren und sich ausbreiten, adhärieren die C-Klone nur sehr schlecht und nehmen eine rundliche Form an. Die C-Klone wachsen klümpchenartig und schwimmen größtenteils als losgelöste Sphäroide im Medium. Eine Färbung mit Trypan-Blau zeigt, dass diese losgelösten Zellen jedoch vital und damit teilungsfähig sind. Im Ergebnis induziert die Flamingo Proteinteilsequenz (SEQ-ID 36) einen veränderten Phänotyp gegenüber den Kontrollzellen.

Figur 7 zeigt eine Darstellung unterschiedlicher Phänotypen der stabil transifizierten HEK293 Zellen. Es sind die unterschiedlichen Phänotypen der mit dem Leervektor (Mock) transfizierten HEK293 Zellen (Mock-Kl; links) und der mit dem Flamingo-C-terminalen Fragment (SEQ-ID 35, SEQ-ID 36) (C-K8; rechts) zu sehen. Während der Mock-Klon gut an der Oberfläche der Kulturschale haftet und seinen Zellkörper ausbreitet, wächst der C-K8-Klon nicht an der Kulturschale an, sondern die Zellen schwimmen als losgelöste Sphäroide im Medium. Beide Klonpopulationen sind jedoch vital.

### Beispiel 4: Adhäsion

Wie zuvor bereits beschrieben schwimmen die C-Klone größtenteils als losgelöste Sphäroide im Medium. Mit Hilfe eines Adhäsionsassays wurde die verringerte Adhäsion der C-Klone quantitativ ausgewertet. Die C-Klone und die Mock-Klone werden hierfür auf mit Fibonektin beschichteten Platten und unbeschichteten Platten in gleicher Zellzahl ausplattiert und über Nacht inkubiert. Mit Kristallviolett (CV) bzw. MTS [3-(4,5-dimethylthiazol-2-yl)-5-(3-carboxymethoxyphenyl)-2-(4-sllfophenyl)-2 H-tetrazolium] (Promega) wurde die Vitalität bzw. die mitochondriale Aktivität gemessen, so dass ermittelt werden konnte, unter welchen Adhäsionsbedingungen die Zellen besser haften und ob die Zellen lebensfähig waren.

### Beispiel 5: Jun-Kinase-Assay

Für die Transfektion in HEK293 Zellen wurden 1 pg pFR-Luc Reporter Plasmid, 50 ng Fusion Transactivator (pFAR-c-jun), 100ng JNK Expressionsvektor und 200 ng Renilla-Luciferase (Transfektion Effizienz) miteinander vermischt. Dazu wurden jeweils 1-3 pg pSecTag2+V5 enthaltend (SEQ-ID 35) (s.o.) bzw. ein Vektor enthaltend pFc-Mekk oder ein Vektor enthaltend frizzled8 (fz8) als Kontrolle/Stimulator zugegeben. Die Zellen wurden über 18 Stunden inkubiert, danach lysiert und anschließend die Luciferase Werte gemessen.

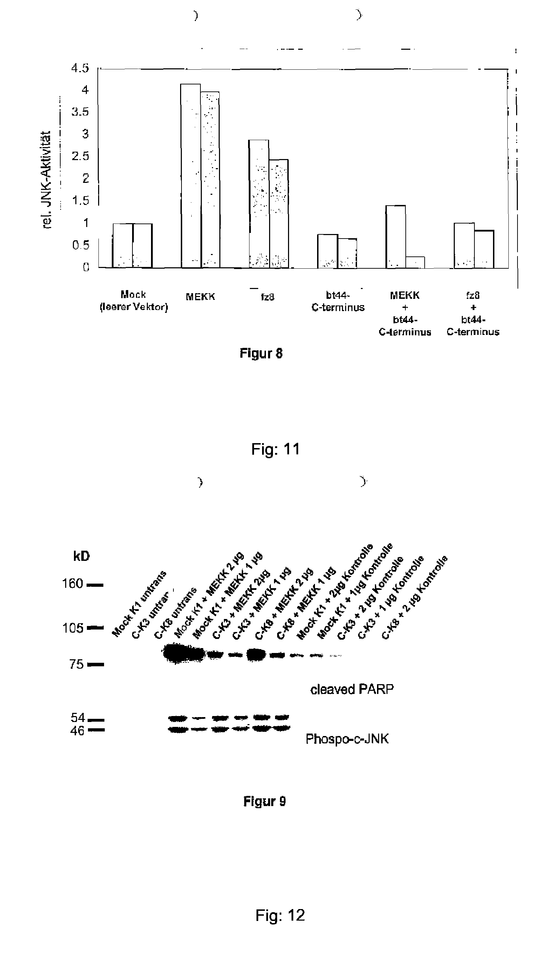

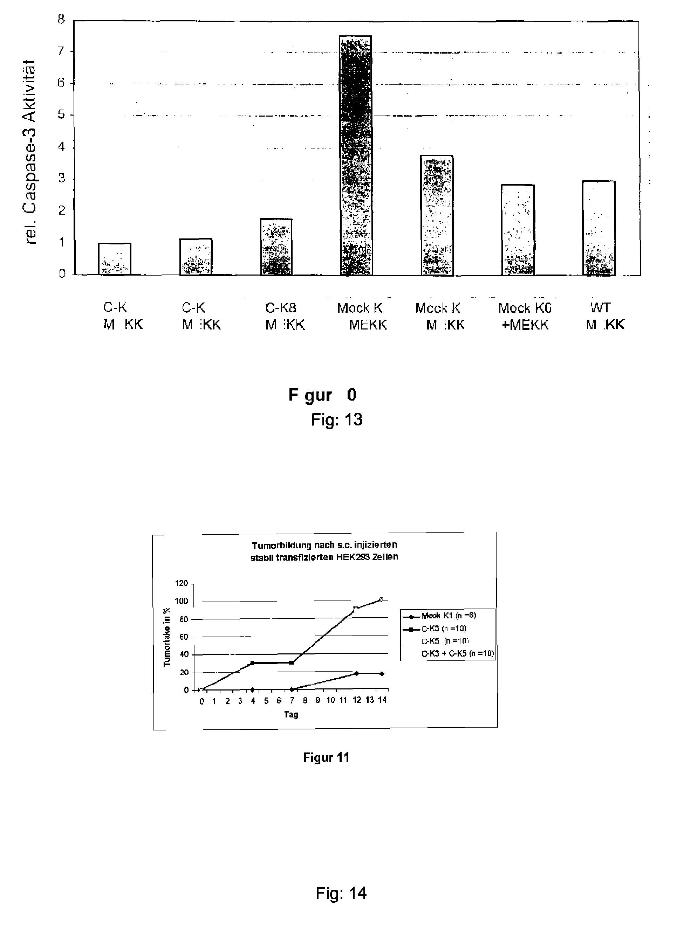

Die Figur 8 zeigt den Einfluss von Flamingo (SEQ-ID 35, 36) auf die JNK-Aktivität in HEK293 Zellen nach einer transienten Transfektion mit MEKK und fz8. MEKK und fz8 stimulieren JNK, wobei MEKK ein stärkerer Stimulator ist als fz8. Der C-terminus von Flamingo allein zeigt verglichen mit der Kontrolle (Leervektor) keinen Effekt. Werden jedoch MEKK oder fz8 mit Flamingo (C-terminus) kotransfiziert fällt die JNK-Aktivität um mehr als die Hälfte ab. Diese Ergebnisse zeigen, dass das C-terminale flamingo-Protein Fragment (SEQ-ID 36) einen hemmenden Effekt auf die Aktivität der durch MEKK oder fz8 stimulierten JNK hat. Gemessen wurde für die Egebnisse der Figur 8 jeweils die relative JNK-Aktivität in transient transfizierten HEK293 Zellen. Es wurden jeweils Doppelwerte gemessen. Als interner Standard wurden die Zellen herangezogen, die mit dem Leervektor (Mock) transfiziert wurden und deren JNK-Aktivität als 1 festgesetzt wurde. Im Ergebnis zeigt die Figur 8, dass die JNK-Aktivität in Zellen, die mit Flamingo kotransfiziert werden, eine im Durchschnitt um den Faktor 5 geringere Aktivität aufweisen, als die Zellen, die mit dem JNK-Aktivator MEKK oder fz8 alleine transfiziert werden. Flamingo zeigt hiermit eine hemmende Wirkung auf die JNK-Aktivität.

### Beispiel 6: Apoptose

Lisovsky et al. konnte zeigen, dass die Aktivierung von JNK im PCP-Signaltransduktionsweg in Xenopus Embryos zur Apoptose führt (Lisovsky et al. 2002, Curr Biol 12(1):53-8). Neu ist, dass die Inhibition von JNK durch Flamingo (SEQ-ID 36) in humanen Krebszelllinien und in humanen Tumoren die Zellen vor Apoptose

schützt, so dass sie einen Wachstumsvorteil gegenüber Zellen, die kein Flamingo exprimieren, erlangen. Dies wird im folgenden beschrieben.

Es konnte gezeigt werden, dass die Stimulierung von JNK durch MEKK in HEK293 zur Apoptose führt. Die auf diese Weise ausgelöste Apoptose kann durch die Flamingo Teilsequenz (SEQ-ID 35, SEQ-ID 36) unterdrückt werden. Der Nachweis wurde im Westernblot und mit dem Apo-ONE® Homogeneous Caspase-3/7 Assay (Promega) erbracht.

Western Blot: Im Westernblot wurden die stabil transfizierten HEK293 Klone HEK-Flamingo-C-terminaler Bereich (SEQ-ID 35, SEQ-ID 36), Mock's (Leervektor) und Wildtyp Zellen untersucht. Diese stabilen HEK293 Zellen wurden mit MEKK transfiziert, um die JNK zu stimulieren. Für die Transfektion wurde Lipofectamine® 2000 (Invitrogen) gemäß den Herstellerangaben verwendet (2,5 µl LF : 1 µg DNA), wobei meist 2 µg pFc-MEKK transfiziert wurden. Um eine Konzentrationsabhängigkeit aufzuzeigen, wurde in einigen Fällen auch 1 µg pFc-MEKK transfiziert. 24 Stunden nach der Transfektion wurden NP40-Proteinlysate von den Zellen hergestellt und zur Western Blot Analyse eingesetzt. Der primäre Antikörper (polyklonaler Ak aus Kaninchen) ist gegen das Cleaved PARP (Poly- ADP-Ribose-Polymerase) gerichtet. Der sekundäre Antikörper ist ein Anti-Kaninchen Antikörper, der an die "Horse Raddish Peroxidase" (HRP) gekoppelt ist. Die Visualisierung der gebundenen Antikörper erfolgte mit dem ECL® System nach Angaben des Herstellers (Amersham Bioscience). In Figur 9 sind die Ergebnisse des Western-Blots gezeigt. Es wurden ein anti-Phospho-SAPK/JNK Antikörper (Cell Signaling; polyklonal aus Kaninchen) zum Nachweis der phosphorylierten JNK und ein anti-cleaved PARP Antikörper (Cell Signaling, polyklonal aus Kaninchen) zum Nachweis der geschnittenen PARP verwendet. Die 116 kDa große PARP scheint an der DNA-Reparatur beteiligt zu sein. Die Caspase-3, eines der Hauptenzyme während der Apoptose, schneidet PARP in ein 24 kDa bzw. 89 kDa großes Fragment. Geschnittene PARP erleichtert den Zellabbau und dient daher als Marker für Apoptose. Aus Figur 9 geht hervor, dass alle Klone, die MEKK exprimieren, ebenfalls eine aktivierte JNK koexprimieren. Die Stimulierung der Kinase durch MEKK resultiert in den Mocks in einer starken Apoptoseantwort und die Zellen sterben. In den Flamingo-Klonen (Flamingo-C-terminales Fragment, SEQ-ID 35, SEQ-ID 36) führt die gleiche Menge MEKK zu einer vielfach geringeren Apoptoseantwort gegenüber den Mock-transfizierten Klonen. Das C-terminale Fragment von Flamingo (SEQ-ID 35, SEQ-ID 36) schützt somit die Zellen vor Apoptose. Für den Tumor, in dem Flamingo überexprimiert wird, bietet eine erniedrigte Apoptoserate einen Wachstumsvorteil.

Zur Figur 9 ist im Einzelnen folgendes anzumerken. Sie stellt einen Westernblot dar, der die Proteinmenge von "cleaved" PARP und die Menge der aktivierten (phosphorylierten) JNK in den erwarteten Größen zeigt. Als Negativkontrolle wurden untransfizierte Mock Klon 1 (Spur 1) bzw. die Klone C-K3 und C-K8 (Spur 2 und 3) und mit einem Kontrollplasmid transfizierte Mock K1 bzw. C-K3 und C-K8 Lysate (Spuren 10 - 14) aufgetragen. Die Transfektion von MEKK in die Zellen führt zur Aktivierung von JNK (Spur 4-9). "Cleaved PARP" dient als Apoptose-Marker. Der MEKK-transfizierte Mock K1 zeigt eine hohe Expression von cleaved PARP (Spur 4, 5), während die MEKK-transfizierten stabilen Flamingo Klone (Spur 6, 7, 8, 9) wesentlich geringere Mengen an cleaved PARP aufweisen, d.h. Flamingo schützt vor Apoptose.

### Beispiel 7: Apo-ONE® Homogeneous Caspase-3/-7 Assay

Der Apo-ONE® Assay (Promega) wurde 24 Stunden nach MEKK-Transfektion in die stabilen HEK293 Zellen gemäß Herstellerangabe durchgeführt. Das Ergebnis ist in der Figur 10 dargestellt. Die stabil mit den Flamingo-C-terminalen Fragment (SEQ-ID 35, SEQ-ID 36) transfizierten HEK293 Klone C-K3, C-K5 und C-K8 zeigen in Anwesenheit von MEKK eine sehr geringe Caspase-3/-7 Aktivität auf, die um den Faktor 4,5 bis 7,5-fach unter der Caspase-3/-7 Aktivität der Kontrolle liegt (Mock K1 + MEKK). Gegenüber den Wildtyp-Zellen, die weder Flamingo noch den leeren Vektor exprimieren, ist die Caspase-3/-7 Aktivität der Klone C-K3, C-K5 und C-K8 um den Faktor 1,7 bis 3 verringert. Alle Ergebnisse wurden auf eine definierte Zellmenge nominiert. Im Ergebnis schützt Flamingo die HEK293 Zellen vor Apoptose und bietet ihnen somit einen Wachstumsvorteil gegenüber den Wildtyp-Zellen.

Figur 10 zeigt im Einzelnen den Einfluß von Flamingo auf die Caspase-3/-7 Aktivität in stabil transfizierteh HEK293 Zellen. Als Negativkontrollen wurden der stabil mit dem Leervektor transfizierte Mock K1 Klon und der HEK293 Wildtyp (nicht transfiziert) verwendet und mit MEKK kotransifiziert. Der JNK-Stimulator MEKK führt zu einer hohen Caspase-3/-7 Aktivität und die Zellen gehen in Apoptose über. Die stabil mit Flamingo transfizierten Zellen zeigen hingegen eine um Faktor 4,5- 7,5 geringere Apoptoserate verglichen mit dem Mock K1 Klon.

Figur 11:

Die stabil transfizierten HEK293 Zellen - Mock-K1 (Vektor ohne Flamingo Insertion), C-K3, C-K5 und ein Gemisch aus C-K3 und C-K5 (Klone mit der C-terminalen Domäne von Flamingo (Seq-ID 35)) wurden in 9 Wochen alte männliche HWi NMRI-nu/nu Mäuse subcutan in die rechte Flanke injiziert. Pro Maus wurden 2 Millionen Zellen in 50 µl PBS-Matrigel im Verhältnis 1:1 verwendet. Das Gemisch (C-K3/K5) bestand aus 1 Million C-K3 und 1 Million C-K5 Zellen in 50 µl PBS-Matrigel. Jede Gruppe beinhaltete 10 Mäuse. Aus der Mock-K1 Kontrollgruppe wurden bei vier Mäusen die Zellen in die subrenale Kapsel injiziert, während die Zellen bei den anderen 6 Mäuse subcutan injiziert wurden. Die subrenale Injektion diente als Positivkontrolle fuer die Viabilitaet der Zellen.

Bei den anderen drei Gruppen (C-K3, C-K5 und C-K3/K5) wurden alle 10 Mäuse subcutan behandelt. Nach 4, 7, 12 und 14 Tagen wurde die Tumorbildung (durch Palpieren) (Figur 1) sowie das Tumorvolumen (durch Ausmessen) (Figur 2) ermittelt.

In Figur 1 ist zu sehen, dass ein großer Anteil der Mäuse, die subcutan mit C-Klonen behandelt wurden, nach 4 Tagen Tumore gebildet haben, während die Mock behandelten Mäuse kaum Tumore aufweisen. Von den C-K3/K5 behandelten Mäusen haben 70% der Mäuse nach 4 Tagen einen Tumor gebildet. Bei den C-K3 und C-K5 behandelten Mäusen beträgt der prozentuale Anteil der Mäuse mit Tumorbildung 30 bzw. 60%. Keine der Mock behandelten Mäuse weist zu diesem Zeitpunkt einen Tumor auf. Auch nach 7 Tagen zeigen alle Mock behandelten Mäuse kein Tumorwachstum, während der Anteil der Mäuse mit Tumorbildung bei den C-K3/K5 behandelten Mäusen auf 90% angestiegen ist. Am Tag 12 bzw. 14 beträgt der Prozentsatz der Mäuse mit Tumorbildung bei den C-Klon behandelten Mäusen in allen Gruppen 90 bis 100%. Zu diesen Zeitpunkten weist lediglich eine von 6 Mock behandelten Mäusen (17%) ein Tumorwachstum auf.

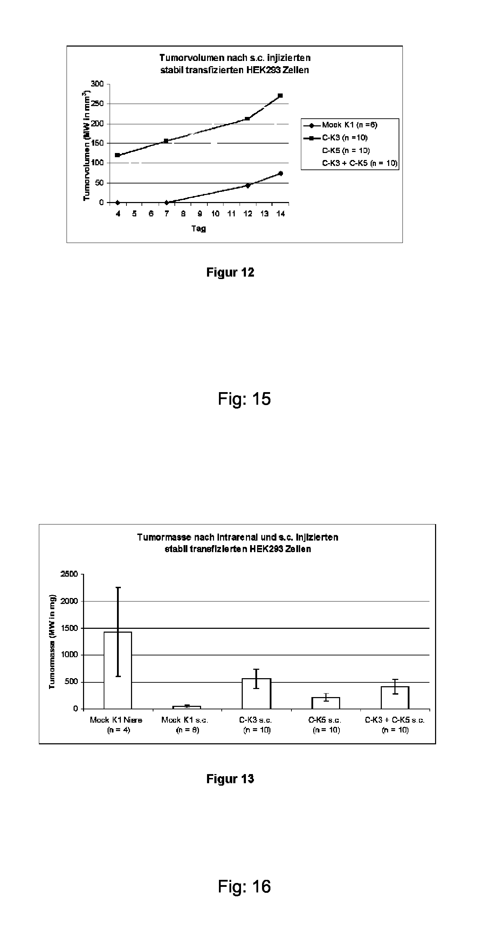

In Figur 12 ist das Tumorvolumen am Tag 4, 7, 12 und 14 nach subcutaner Injektion der stabilen Klone (Mock-K1, C-K3, C-K5 und C-K3/K5) in die Mäuse dargestellt. Das Tumorvolumen wurde wie folgt ermittelt: Länge und Breite des Tumors wurden von ein und derselben Person waehrend der gesamten Versuchsdauer vermessen, um individuelle Schwankungen auszuschließen. Da der Tumor nur zweidimensional vermessen werden konnte, wurde das Tumorvolumen anschließend nach folgender Formel ermittelt:

### Breite x Breite x Länge .

2

In der Graphik sind die Mittelwerte des Tumorvolumens - vermessen bei jeweils 6 (Mock-K1) bzw. 10 Mäusen (C-Klone) pro Gruppe - dargestellt. Wie bereits erwähnt zeigten die Mock behandelten Mäuse kein Tumorwachstum am Tag 4 und 7. Am Tag 12 und 14 konnte ein Tumorwachstum bei den Mock behandelten Mäusen ermittelt werden. Das maximal erreichte Tumorvolumen betrug 74 mm³. Bei den C-Klon behandelten Mäusen konnte am Tag 4 ein Tumorvolumen von 74 bis 118 mm³ in den einzelnen Gruppen ermittelt werden. Bis zum Tag 14 stieg das Tumorvolumen hier auf Werte von 206 bis 270 mm³.

In Figur 13 ist die Tumormasse am Tag 14 nach intrarenaler und subcutaner Injektion der stabilen Klone (Mock-K1, C-K3, C-K5 und C-K3/K5) in die Mäuse dargestellt. Am Tag 14 wurden die Tumore aus den Mäusen operativ entfernt und gewogen.

Die Mock-K1 HEK293 Zellen führten in der Niere zu einen Tumor mit einer Masse von 1427 mg. Das gute Anwachsen dieser aus embryonalen Nierenzellen abstammenden HEK293 Zellen in der Niere bestätigt die Vitalität der injizierten Mock-K1 Zellen. Obwohl diese Mock-K1 Zellen vital genug sind, um Tumore in der Niere zu bilden, zeigen sie subcutan injiziert lediglich ein mittleres Tumorgewicht von 41 mg. Dagegen weisen die Tumore entstanden durch subcutane Injektion von C-K Zellen, ein Gewicht von 217 bis 560 mg auf.

Im Ergebnis zeigen die Figuren 1-3, dass das stabil transfizierte C-terminale Fragment von Flamingo im Mausmodell einen Wachstumsvorteil gegenüber den Mock-Klonen besitzt.

Sequenzprotokoll - freier Text

>SEQID1_AF231024.1

ATGGCGCCGCCGCCGCCGCCCGTGCTGCCCGTGCTGCTGCTCCTGG

CCGCCGCCGCCGCCCTGCCGGCGA

TGGGGCTGCGAGCGGCCGCCTGGGAGCCGCGCGTACCCGGCGGGAC

CCGCGCCTTCGCCCTCCGGCCCGG

CTGTACCTACGCGGTGGGCGCCGCTTGCACGCCCCGGGCGCCGCGG

GAGCTGCTGGACGTGGGCCGCGAT

GGGCGGCTGGCAGGACGTCGGCGCGTCTCGGGCGCGGGGCGCCCG

CTGCCGCTGCAAGTCCGCTTGGTGG

CCCGCAGTGCCCCGACGGCGCTGAGCCGCCGCCTGCGGGCGCGCAC

GCACCTTCCCGGCTGCGGAGCCCG

TGCCCGGCTCTGCGGAACCGGTGCCCGGCTCTGCGGGGCGCTCTGC

TTCCCCGTCCCCGGCGGCTGCGCG

GCCGCGCAGCATTCGGCGCTCGCAGCTCCGACCACCTTACCCGCCTG

CCGCTGCCCGCCGCGCCCCAGGC

CCCGCTGTCCCGGCCGTCCCATCTGCCTGCCGCCGGGCGGCTCGGT
CCGCCTGCGTCTGCTGTGCGCCCT
GCGGCGCGCGGCTGGCGCCGTCCGGGTGGGACTGGCGCTGGAGGC
CGCCACCGCGGGGACGCCCTCCGCG
TCGCCATCCCCATCGCCGCCCCTGCCGCCGAACTTGCCCGAAGCCCG
GGCGGGGCCGGCGCGACGGGCCC
GGCGGGGCACGAGCGGCAGAGGGAGCCTGAAGTTTCCGATGCCCAA
CTACCAGGTGGCGTTGTTTGAGAA
CGAACCGGCGGGCACCCTCATCCTCCAGCTGCACGCGCACTACACCA
TCGAGGGCGAGGAGGAGCGCGTG
AGCTATTACATGGAGGGGCTGTTCGACGAGCGCTCCCGGGGCTACTT
CCGAATCGACTCTGCCACGGGCG
CCGTGAGCACGGACAGCGTACTGGACCGCGAGACCAAGGAGACGCAC
GTCCTCAGGGTGAAAGCCGTGGA
CTACAGTACGCCGCCGCGCTCGGCCACCACCTACATCACTGTCTTGGT
CAAAGACACCAACGACCACAGC
CCGGTCTTCGAGCAGTCGGAGTACCGCGAGCGCGTGCGGGAGAACCT
GGAGGTGGGCTACGAGGTGCTGA
CCATCCGCGCCAGCGACCGCGACTCGCCCATCAACGCCAACTTGCGT
TACCGCGTGTTGGGGGGCGCGTG

GGACGTCTTCCAGCTCAACGAGAGCTCTGGCGTGGTGAGCACACGGG
CGGTGCTGGACCGGGAGGAGGCG
GCCGAGTACCAGCTCCTGGTGGAGGCCAACGACCAGGGGCGCAATCC
GGGCCCGCTCAGTGCCACGGCCA
CCGTGTACATCGAGGTGGAGGACGAGAACGACAACTACCCCCAGTTC
AGCGAGCAGAACTACGTGGTCCA
GGTGCCCGAGGACGTGGGGCTCAACACGGCTGTGCTGCGAGTGCAG
GCCACGGACCGGGACCAGGGCCAG
AACGCGGCCATTCACTACAGCATCCTCAGCGGGAACGTGGCCGGCCA
GTTCTACCTGCACTCGCTGAGCG
GGATCCTGGATGTGATCAACCCCTTGGATTTCGAGGATGTCCAGAAAT
ACTCGCTGAGCATTAAGGCCCA
GGATGGGGGCCGGCCCCCGCTCATCAATTCTTCAGGGGTGGTGTCTG
TGCAGGTGCTGGATGTCAACGAC
AACGAGCCTATCTTTGTGAGCAGCCCCTTCCAGGCCACGGTGCTGGA
GAATGTGCCCCTGGGCTACCCCG
TGGTGCACATTCAGGCGGTGGACGCGGACTCTGGAGAGAACGCCCGG
CTGCACTATCGCCTGGTGGACAC
GGCCTCCACCTTTCTGGGGGGCGGCAGCGCTGGGCCTAAGAATCCTG
CCCCCACCCCTGACTTCCCCTTC

CAGATCCACAACAGCTCCGGTTGGATCACAGTGTGTGCCGAGCTGGA
CCGCGAGGAGGTGGAGCACTACA
GCTTCGGGGTGGAGGCGGTGGACCACGGCTCGCCCCCATGAGCTC
CTCCACCAGCGTGTCCATCACGGT
GCTGGACGTGAATGACAACGACCCGGTGTTCACGCAGCCCACCTACG
AGCTTCGTCTGAATGAGGATGCG
GCCGTGGGGAGCAGCGTGCTGACCCTGCAGGCCCGCGACCGTGACG
CCAACAGTGTGATTACCTACCAGC
TCACAGGCGGCAACACCCGGAACCGCTTTGCACTCAGCAGCCAGAGA
GGGGGCGGCCTCATCACCCTGGC
GCTACCTCTGGACTACAAGCAGGAGCAGCAGTACGTGCTGGCGGTGA
CAGCATCCGACGGCACACGGTCG
CACACTGCGCATGTCCTAATCAACGTCACTGATGCCAACACCCACAGG
CCTGTCTTTCAGAGCTCCCATT
ACACAGTGAGTGTCAGTGAGGACAGGCCTGTGGGCACCTCCATTGCTA
CCCTCAGTGCCAACGATGAGGA
CACAGGAGAGAATGCCCGCATCACCTACGTGATTCAGGACCCCGTGC
CGCAGTTCCGCATTGACCCCGAC
AGTGGCACCATGTACACCATGATGGAGCTGGACTATGAGAACCAGGTC
GCCTACACGCTGACCATCATGG

CCCAGGACAACGGCATCCCGCAGAAATCAGACACCACCACCCTAGAG
ATCCTCATCCTCGATGCCAATGA

CAATGCACCCCAGTTCCTGTGGGATTTCTACCAGGGTTCCATCTTTGA
GGATGCTCCACCCTCGACCAGC

ATCCTCCAGGTCTCTGCCACGGACCGGGACTCAGGTCCCAATGGGCG
TCTGCTGTACACCTTCCAGGGTG

GGGACGACGGCGATGGGGACTTCTACATCGAGCCCACGTCCGGTGTG
ATTCGCACCCAGCGCCGGCTGGA

CCGGGAGAATGTGGCCGTGTACAACCTTTGGGCTCTGGCTGTGGATC
GGGGCAGTCCCACTCCCCTTAGC

GCCTCGGTAGAAATCCAGGTGACCATCTTGGACATTAATGACAATGCC
CCCATGTTTGAGAAGGACGAAC

TGGAGCTGTTTGTTGAGGAGAACAACCCAGTGGGGTCGGTGGTGGCA
AAGATTCGTGCTAACGACCCTGA

TGAAGGCCCTAATGCCCAGATCATGTATCAGATTGTGGAAGGGGACAT
GCGGCATTTCTTCCAGCTGGAC

CTGCTCAACGGGGACCTGCGTGCCATGGTGGAGCTGGACTTTGAGGT
CCGGCGGGAGTATGTGCTGGTGG

TGCAGGCCACGTCGGCTCCGCTGGTGAGCCGAGCCACGGTGCACATC
CTTCTCGTGGACCAGAATGACAA

CCCGCCTGTGCTGCCCGACTTCCAGATCCTCTTCAACAACTATGTCAC
CAACAAGTCCAACAGTTTCCCC

ACCGGCGTGATCGGCTGCATCCCGGCCCATGACCCCGACGTGTCAGA
CAGCCTCAACTACACCTTCGTGC

AGGGCAACGAGCTGCGCCTGTTGCTGCTGGACCCCGCCACGGGCGAA
CTGCAGCTCAGCCGCGACCTGGA

CAACAACCGGCCGCTGGAGGCGCTCATGGAGGTGTCTGTGTCTGATG
GCATCCACAGCGTCACGGCCTTC

TGCACCCTGCGTGTCACCATCATCACGGACGACATGCTGACCAACAGC
ATCACTGTCCGCCTGGAGAACA

TGTCCCAGGAGAAGTTCCTGTCCCCGCTGCTGGCCCTCTTCGTGGAG
GGGGTGGCCGCCGTGCTGTCCAC

CACCAAGGACGACGTCTTCGTCTTCAACGTCCAGAACGACACCGACGT
CAGCTCCAACATCCTGAACGTG

ACCTTCTCGGCGCTGCTGCCTGGCGGCGTCCGCGGCCAGTTCTTCCC
GTCGGAGGACCTGCAGGAGCAGA

TCTACCTGAATCGGACGCTGCTGACCACCATCTCCACGCAGCGCGTGC
TGCCCTTCGACGACAACATCTG

CCTGCGCGAGCCCTGCGAGAACTACATGAAGTGCGTGTCCGTTCTGC
GATTCGACAGCTCCGCGCCCTTC

CTCAGCTCCACCACCGTGCTCTTCCGGCCCATCCACCCCATCAACGGC
CTGCGCTGCCGCTGCCCGCCCG
GCTTCACCGGCGACTACTGCGAGACGGAGATCGACCTCTGCTACTCC
GACCCGTGCGGCGCCAACGGCCG
CTGCCGCAGCCGCGAGGGCGGCTACACCTGCGAGTGCTTCGAGGACT
TCACTGGAGAGCACTGTGAGGTG
GATGCCCGCTCAGGCCGCTGTGCCAACGGGGTGTGCAAGAACGGGG
GCACCTGCGTGAACCTGCTCATCG
GCGGCTTCCACTGCGTGTGTCCTCCTGGCGAGTATGAGAGGCCCTAC
TGTGAGGTGACCACCAGGAGCTT
CCCGCCCCAGTCCTTCGTCACCTTCCGGGGCCTGAGACAGCGCTTCC
ACTTCACCATCTCCCTCACGTTT
GCCACTCAGGAAAGGAACGGCTTGCTTCTCTACAACGGCCGCTTCAAT
GAGAAGCACGACTTCATCGCCC
TGGAGATCGTGGACGAGCAGGTGCAGCTCACCTTCTCTGCAGGCGAG
ACAACAACGACCGTGGCACCGAA
GGTTCCCAGTGGTGTGAGTGACGGGCGGTGGCACTCTGTGCAGGTGC
AGTACTACAACAAGCCCAATATT
GGCCACCTGGGCCTGCCCCATGGGCCGTCCGGGGAAAAGATGGCCG
TGGTGACAGTGGATGATTGTGACA

CAACCATGGCTGTGCGCTTTGGAAAGGACATCGGGAACTACAGCTGC
GCTGCCCAGGGCACTCAGACCGG
CTCCAAGAAGTCCCTGGATCTGACCGGCCCTCTACTCCTGGGGGGTG
TCCCCAACCTGCCAGAAGACTTC
CCAGTGCACAACCGGCAGTTCGTGGGCTGCATGCGGAACCTGTCAGT
CGACGGCAAAAATGTGGACATGG
CCGGATTCATCGCCAACAATGGCACCCGGGAAGGCTGCGCTGCTCGG
AGGAACTTCTGCGATGGGAGGCG
GTGTCAGAATGGAGGCACCTGTGTCAACAGGTGGAATATGTATCTGTG
TGAGTGTCCACTCCGATTCGGC
GGGAAGAACTGTGAGCAAGCCATGCCTCACCCCCAGCTCTTCAGCGG
TGAGAGCGTCGTGTCCTGGAGTG
ACCTGAACATCATCATCTCTGTGCCCTGGTACCTGGGGCTCATGTTCC
GGACCCGGAAGGAGGACAGCGT
TCTGATGGAGGCCACCAGTGGTGGGCCCACCAGCTTTCGCCTCCAGA
TCCTGAACAACTACCTCCAGTTT
GAGGTGTCCCACGGCCCCTCCGATGTGGAGTCCGTGATGCTGTCCGG
GTTGCGGGTGACCGACGGGGAGT
GGCACCACCTGCTGATCGAGCTGAAGAATGTTAAGGAGGACAGTGAG
ATGAAGCACCTGGTCACCATGAC

CTTGGACTATGGGATGGACCAGAACAAGGCAGATATCGGGGGCATGC
TTCCCGGGCTGACGGTAAGGAGC
GTGGTGGTCGGAGGCGCCTCTGAAGACAAGGTCTCCGTGCGCCGTGG
ATTCCGAGGCTGCATGCAGGGAG
TGAGGATGGGGGGGACGCCCACCAACGTCGCCACCCTGAACATGAAC
AACGCACTCAAGGTCAGGGTGAA
GGACGGCTGTGATGTGGACGACCCCTGTACCTCGAGCCCCTGTCCCC
CCAATAGCCGCTGCCACGACGCC
TGGGAGGACTACAGCTGCGTCTGTGACAAAGGGTACCTTGGAATAAAC
TGTGTGGATGCCTGTCACCTGA
ACCCCTGCGAGAACATGGGGGCCTGCGTGCGCTCCCCCGGCTCCCC
GCAGGGCTACGTGTGCGAGTGTGG
GCCCAGTCACTACGGGCCGTACTGTGAGAACAAACTCGACCTTCCGTG
CCCCAGAGGCTGGTGGGGAAC
CCCGTCTGTGGACCCTGCCACTGTGCCGTCAGCAAAGGCTTTGATCCC
GACTGTAATAAGACCAACGGCC
AGTGCCAATGCAAGGAGAATTACTACAAGCTCCTAGCCCAGGACACCT
GTCTGCCCTGCGACTGCTTCCC
CCATGGCTCCCACAGCCGCACTTGCGACATGGCCACCGGGCAGTGTG
CCTGCAAGCCCGGCGTCATCGGC

CGCCAGTGCAACCGCTGCGACAACCCGTTTGCCGAGGTCACCACGCT
CGGCTGTGAAGTGATCTACAATG
GCTGTCCCAAAGCATTTGAGGCCGGCATCTGGTGGCCACAGACCAAG
TTCGGGCAGCCGGCTGCGGTGCC
ATGCCCTAAGGGATCCGTTGGAAATGCGGTCCGACACTGCAGCGGGG
AGAAGGGCTGGCTGCCCCCAGAG
CTCTTTAACTGTACCACCATCTCCTTCGTGGACCTCAGGGCCATGAATG
AGAAGCTGAGCCGCAATGAGA
CGCAGGTGGACGGCGCCAGGGCCCTGCAGCTGGTGAGGGCGCTGCG
CAGTGCTACACAGCACACGGGCAC
GCTCTTTGGCAATGACGTGCGCACGGCCTACCAGCTGCTGGGCCACG
TCCTTCAGCACGAGAGCTGGCAG
CAGGGCTTCGACCTGGCAGCCACGCAGGACGCCGACTTTCACGAGGA
CGTCATCCACTCGGGCAGCGCCC
TCCTGGCCCCAGCCACCAGGGCGGCGTGGGAGCAGATCCAGCGGAG
CGAGGGCGGCACGGCACAGCTGCT
CCGGCGCCTCGAGGGCTACTTCAGCAACGTGGCACGCAACGTGCGGC
GGACGTACCTGCGGCCCTTCGTC
ATCGTCACCGCCAACATGATTCTTGCTGTCGACATCTTTGACAAGTTCA
ACTTTACGGGAGCCAGGGTCC

CGCGATTCGACACCATCCATGAAGAGTTCCCCAGGGAGCTGGAGTCCT
CCGTCTCCTTCCCAGCCGACTT
CTTCAGACCACCTGAAGAAAAGAAGGCCCCCTGCTGAGGCCGGCTG
GCCGGAGGACCACCCCGCAGACC
ACGCGCCCGGGGCCTGGCACCGAGAGGGAGGCCCCGATCAGCAGGC
GGAGGCGACACCCTGATGACGCTG
GCCAGTTCGCCGTCGCTCTGGTCATCATTTACCGCACCCTGGGGCAG
CTCCTGCCCGAGCGCTACGACCC
CGACCGTCGCAGCCTCCGGTTGCCTCACCGGCCCATCATTAATACCCC
GATGGTGAGCACGCTGGTGTAC
AGCGAGGGGGCTCCGCTCCCGAGACCCCTGGAGAGGCCCGTCCTGG
TGGAGTTCGCCCTGCTGGAGGTGG
AGGAGCGAACCAAGCCTGTCTGCGTGTTCTGGAACCACTCCCTGGCC
GTTGGTGGGACGGGAGGGTGGTC
TGCCCGGGGCTGCGAGCTCCTGTCCAGGAACCGGACACATGTCGCCT
GCCAGTGCAGCCACACAGCCAGC
TTTGCGGTGCTCATGGATATCTCCAGGCGTGAGAACGGGGAGGTCCT
GCCTCTGAAGATTGTCACCTATG
CCGCTGTGTCCTTGTCACTGGCAGCCCTGCTGGTGGCCTTCGTCCTCC
TGAGCCTGGTCCGCATGCTGCG

CTCCAACCTGCACAGCATTCACAAGCACCTCGCCGTGGCGCTCTTCCT

CTCTCAGCTGGTGTTCGTGATT

GGGATCAACCAGACGGAAAACCCGTTTCTGTGCACAGTGGTTGCCATC

CTCCTCCACTACATCTACATGA

GCACCTTTGCCTGGACCCTCGTGGAGAGCCTGCATGTCTACCGCATGC

TGACCGAGGTGCGCAACATCGA

CACGGGGCCCATGCGGTTCTACTACGTCGTGGGCTGGGGCATCCCGG

CCATTGTCACAGGACTGGCGGTC

GGCCTGGACCCCCAGGGCTACGGGAACCCCGACTTCTGCTGGCTGTC

GCTTCAAGACACCCTGATTTGGA

GCTTTGCGGGGCCCATCGGAGCTGTTATAATCATCAACACAGTCACTT

CTGTCCTATCTGCAAAGGTTTC

CTGCCAAAGAAAGCACCATTATTATGGAAAAAGGGATCGTCTCCCT

GCTGAGGACCGCATTCCTCCTG

CTGCTGCTCATCAGCGCCACCTGGCTGCTGGGGCTGCTGGCTGTGAA

CCGCGATGCACTGAGCTTTCACT

ACCTCTTCGCCATCTTCAGCGGCTTACAGGGCCCCTTCGTCCTCCTTTT

CCACTGCGTGCTCAACCAGGA

GGTCCGGAAGCACCTGAAGGGCGTGCTCGGCGGGAGGAAGCTGCAC

CTGGAGGACTCCGCCACCACCAGG

GCCACCCTGCTGACGCGCTCCCTCAACTGCAACACCACCTTCGGTGAC
GGGCCTGACATGCTGCGCACAG
ACTTGGGCGAGTCCACCGCCTCGCTGGACAGCATCGTCAGGGATGAA
GGGATCCAGAAGCTCGGCGTGTC
CTCTGGGCTGGTGAGGGGCAGCCACGGAGAGCCAGACGCGTCCCTC
ATGCCCAGGAGCTGCAAGGATCCC
CCTGGCCACGATTCCGACTCAGATAGCGAGCTGTCCCTGGATGAGCA
GAGCAGCTCTTACGCCTCCTCAC
ACTCGTCAGACAGCGAGGACGATGGGGTGGAGCTGAGGAAAAATGG
GACCCGGCCAGGGGCGCCGTCCA
CAGCACCCCCAAAGGGGACGCTGTGGCCAACCACGTTCCGGCCGGCT
GGCCCGACCAGAGCCTGGCTGAG
AGTGACAGTGAGGACCCCAGCGGCAAGCCCCGCCTGAAGGTGGAGAC
CAAGGTCAGCGTGGAGCTGCACC
GCGAGGAGCAGGGCAGTCACCGTGGAGAGTACCCCCCGGACCAGGA
GAGCGGGGGCGCAGCCAGGCTTGC
TAGCAGCCAGCCCCCAGAGCAGAGGAAAGGCATCTTGAAAAATAAAGT
CACCTACCCGCCGCCGCTGACG
CTGACGGAGCAGACGCTGAAGGGCCGGCTCCGGGAGAAGCTGGCCG
ACTGTGAGCAGAGCCCCACATCCT

CGCGCACGTCTTCCCTGGGCTCTGGCGGCCCCGACTGCGCCATCACA
GTCAAGAGCCCTGGGAGGGAGCC
GGGGCGTGACCACCTCAACGGGGTGGCCATGAATGTGCGCACTGGGA
GCGCCCAGGCCGATGGCTCCGAC
TCTGAGAAACCGTGAGGCAAGCCCGTCACCCCACACAGGCTGCGGCA
TCACCCTCAGACCTTGGAGCCCA
AGGGGCCACTGCCCTTGAAGTGGAGTGGCCCAGAGTGTGGCGGTCC
CCATGGTGGCAGCCCCCGACTG
ATCATCCAGACACAAAGGTCTTGGTTCTCCCAGGAGCTCAGGGCCTGT
CAGACCTGGTGACAAGTGCCAA
AGGCCACAGGCATGAGGGAGGCGTGGACCACTGGGCCAGCACCGCT
GAGTCCTAAGACTGCAGTCAAAGC
CAGAACTGAGAGGGGACCCCAGACTGGGCCCAGAGGCTGGCCAGAG
TTCAGGAACGCCGGGCACAGACCA
AAGACCGCGGTCCAGCCCCGCCCAGGCGGGCATCTCATGGCAGTGC
GGACCCGTGGCTGGCAGCCCGGGC
AGTCCTTTGCAAAGGCACCCCTTGTCTTAAAATCACTTCGCTATGTGGG
AAAGGTGGAGATACTTTTATA
TATTTGTATGGGACTCTGAGGAGGTGCAACCTGTATATATATTGCATTC
GTGCTGACTTTGTTATCCCGA

GAGATCCATGCAATGATCTCTTGCTGTCTTCTCTGTCAAGATTGCACAG
TTGTACTTGAATCTGGCATGT
GTTGACGAAACTGGTGCCCCAGCAGATCAAAGGTGGGAAATACGTCAG
CAGTGGGGCTAAAACCAAGCGG
CTAGAAGCCCTACAGCTGCCTTCGGCCAGGAAGTGAGGATGGTGTGG
GCCCTCCCCGCCGGCCCCCTGGG
TCCCCAGTGTTCGCTGTGTGTGCGTTTGTCCTCTGCTGCCATCTGCCC
CGGCTGTGTGAATTCAAGACAG
GGCAGTGCAGCACTAGGCAGGTGTGAGGAGCCCTGCTGAGGTCACTG
TGGGGCACGGTTGCCACACGGCT
GTCATTTTTCACCTGGTCATTCTGTGACCACCACCCCCTCCCCTCACCG
CCTCCCAGGTGGCCCGGGAGC
TGCAGGTGGGGATGGCTTTGTCCTTTGCTCCTGCTCCCCGTGGGACCT
GGGACCTTAAAGCGTTGCAGGT
TCCTGATTTGGACAGAGGTGTGGGGCCTTCCAGGCCGTTACATACCTC
CTGCCAATTCTCTAACTCTCTG
AGACTGCGAGGATCTCCAGGCAGGGTTCTCCCCTCTGGAGTCTGACC
AATTACTTCATTTTGCTTCAAAT
GGCCAATTGTGCAGAGGGACAAAGCCACAGCCACACTCTTCAACGGTT
ACCAAACTGTTTTTGGAAATTC

ACACCAAGGTCGGGCCCACTGCAGGCAGCTGGCACAGCGTGGCCCG
AGGGGCTGTGGAACGGGTCCCGGA
ACTGTCAGACATGTTTGATTTTAGCGTTTCCTTTGTTCTTCAAATCAGGT
GCCCAAATAAGTGATCAGCA
CAGCTGCTTCCAAATAGGAGAAACCATAAAATAGGATGAAAATCAAGTA
AAATGCAAAGATGTCCACACT
GTTTTAAACTTGACCCTGATGAAAATGTGAGCACTGTTAGCAGATGCCT
ATGGGAGAGGAAAAGCGTATC
TGAAAATGGTCCAGGACAGGAGGATGAAATGAGATCCCAGAGTCCTCA
CACCTGAATGAATTATACATGT
GCCTTACCAGGTGAGTGGTCTTTCGAAGATAAAAACTCTAGTCCCTTT
AAACGTTTGCCCCTGGCGTTT
CCTAAGTACGAAAAGGTTTTTAAGTCTTCGAACAGTCTCCTTTCATGAC
TTTAACAGGATTCTGCCCCCT
GAGGTGTAATTTTTTTGTTCTATTTTTTTCCACGTACTCCACAGCCAACA
TCACGAGGTGTAATTTTTAA
TTTGATCAGAACTGTTACCAAAAAACAACTGTCAGTTTTATTGAGATGG
GAAAAATGTAAACCTATTTTT
ATTACTTAAGACTTTATGGGAGAGATTAGACACTGGAGGTTTTTAACAG
AACGTGTATTTATTAATGTTC

AAAACACTGGAATTACAAATGAGAAGAGTCTACAATAAATTAAGATTTTT
GAATTTGTACTTCTGCGGTG
CTGGTTTTTCTCCACAAACACCCCCGCCCCTCCCCATGCCCAGGGTGG
CCGTGGAAGGGACGGTTTACGG
ACGTGCAGCTGAGCTGTCCGTGTCCCATGCTCCCTCAGCCAGTGGAA
CGTGCCGGAACTTTTTGTCCATT
CCCTAGTAGGCCTGCCACAGCCTAGATGGGCAGTTTTTGTCTTTCACC
AAATTTGAGGACTTTTTTTTT
TGCCATTATTTCTTCAGTTTTCTTTTCTTGCACTGATCTTTCTCCTCTCCT
TCTGTGACTCCAGTGACTC
AGACGTTAGACCTCTTGATGTTTTCCCACTGGTCCCTGAGGCTCTGTTC
>SEQID2_AAF61930.1
MAPPPPPVLPVLLLLAAAAALPAMGLRAAAWEPRVPGGTRAFALRPGCTY
AVGAACTPRAPRELLDVGRD
GRLAGRRRVSGAGRPLPLQVRLVARSAPTALSRRLRARTHLPGCGARAR
LCGTGARLCGALCFPVPGGCA
AAQHSALAAPTTLPACRCPPRPRPRCPGRPICLPPGGSVRLRLLCALRRA
AGAVRVGLALEAATAGTPSA
SPSPSPPLPPNLPEARAGPARRARRGTSGRGSLKFPMPNYQVALFENEP
AGTLILQLHAHYTIEGEEERV

SYYMEGLFDERSRGYFRIDSATGAVSTDSVLDRETKETHVLRVKAVDYST
PPRSATTYITVLVKDTNDHS
PVFEQSEYRERVRENLEVGYEVLTIRASDRDSPINANLRYRVLGGAWDVF
QLNESSGVVSTRAVLDREEA
AEYQLLVEANDQGRNPGPLSATATVYIEVEDENDNYPQFSEQNYVVQVPE
DVGLNTAVLRVQATDRDQGQ
NAAIHYSILSGNVAGQFYLHSLSGILDVINPLDFEDVQKYSLSIKAQDGGRP
PLINSSGVVSVQVLDVND
NEPIFVSSPFQATVLENVPLGYPVVHIQAVDADSGENARLHYRLVDTASTF
LGGGSAGPKNPAPTPDFPF
QIHNSSGWITVCAELDREEVEHYSFGVEAVDHGSPPMSSSTSVSITVLDV
NDNDPVFTQPTYELRLNEDA
AVGSSVLTLQARDRDANSVITYQLTGGNTRNRFALSSQRGGGLITLALPLD
YKQEQQYVLAVTASDGTRS
HTAHVLINVTDANTHRPVFQSSHYTVSVSEDRPVGTSIATLSANDEDTGEN
ARITYVIQDPVPQFRIDPD
SGTMYTMMELDYENQVAYTLTIMAQDNGIPQKSDTTTLEILILDANDNAPQ
FLWDFYQGSIFEDAPPSTS
ILQVSATDRDSGPNGRLLYTFQGGDDGDGDFYIEPTSGVIRTQRRLDREN
VAVYNLWALAVDRGSPTPLS

ASVEIQVTILDINDNAPMFEKDELELFVEENNPVGSVVAKIRANDPDEGPNA
QIMYQIVEGDMRHFFQLD
LLNGDLRAMVELDFEVRREYVLVVQATSAPLVSRATVHILLVDQNDNPPVL
PDFQILFNNYVTNKSNSFP
TGVIGCIPAHDPDVSDSLNYTFVQGNELRLLLLDPATGELQLSRDLDNNRP
LEALMEVSVSDGIHSVTAF
CTLRVTIITDDMLTNSITVRLENMSQEKFLSPLLALFVEGVAAVLSTTKDDVF
VFNVQNDTDVSSNILNV
TFSALLPGGVRGQFFPSEDLQEQIYLNRTLLTTISTQRVLPFDDNICLREPC
ENYMKCVSVLRFDSSAPF
LSSTTVLFRPIHPINGLRCRCPPGFTGDYCETEIDLCYSDPCGANGRCRSR
EGGYTCECFEDFTGEHCEV
DARSGRCANGVCKNGGTCVNLLIGGFHCVCPPGEYERPYCEVTTRSFPP
QSFVTFRGLRQRFHFTISLTF
ATQERNGLLLYNGRFNEKHDFIALEIVDEQVQLTFSAGETTTTVAPKVPSG
VSDGRWHSVQVQYYNKPNI
GHLGLPHGPSGEKMAVVTVDDCDTTMAVRFGKDIGNYSCAAQGTQTGSK
KSLDLTGPLLLGGVPNLPEDF
PVHNRQFVGCMRNLSVDGKNVDMAGFIANNGTREGCAARRNFCDGRRC
QNGGTCVNRWNMYLCECPLRFG

GKNCEQAMPHPQLFSGESVVSWSDLNIIISVPWYLGLMFRTRKEDSVLME
ATSGGPTSFRLQILNNYLQF
EVSHGPSDVESVMLSGLRVTDGEWHHLLIELKNVKEDSEMKHLVTMTLDY
GMDQNKADIGGMLPGLTVRS
VVVGGASEDKVSVRRGFRGCMQGVRMGGTPTNVATLNMNNALKVRVKD
GCDVDDPCTSSPCPPNSRCHDA
WEDYSCVCDKGYLGINCVDACHLNPCENMGACVRSPGSPQGYVCECGP
SHYGPYCENKLDLPCPRGWWGN
PVCGPCHCAVSKGFDPDCNKTNGQCQCKENYYKLLAQDTCLPCDCFPH
GSHSRTCDMATGQCACKPGVIG
RQCNRCDNPFAEVTTLGCEVIYNGCPKAFEAGIWWPQTKFGQPAAVPCP
KGSVGNAVRHCSGEKGWLPPE
LFNCTTISFVDLRAMNEKLSRNETQVDGARALQLVRALRSATQHTGTLFG
NDVRTAYQLLGHVLQHESWQ
QGFDLAATQDADFHEDVIHSGSALLAPATRAAWEQIQRSEGGTAQLLRRL
EGYFSNVARNVRRTYLRPFV
IVTANMILAVDIFDKFNFTGARVPRFDTIHEEFPRELESSVSFPADFFRPPE
EKEGPLLRPAGRRTTPQT
TRPGPGTEREAPISRRRRHPDDAGQFAVALVIIYRTLGQLLPERYDPDRRS
LRLPHRPIINTPMVSTLVY

SEGAPLPRPLERPVLVEFALLEVEERTKPVCVFWNHSLAVGGTGGWSAR
GCELLSRNRTHVACQCSHTAS
FAVLMDISRRENGEVLPLKIVTYAAVSLSLAALLVAFVLLSLVRMLRSNLHSI
HKHLAVALFLSQLVFVI
GINQTENPFLCTVVAILLHYIYMSTFAWTLVESLHVYRMLTEVRNIDTGPMR
FYYVVGWGIPAIVTGLAV
GLDPQGYGNPDFCWLSLQDTLIWSFAGPIGAVIIINTVTSVLSAKVSCQRK
HHYYGKKGIVSLLRTAFLL
LLLISATWLLGLLAVNRDALSFHYLFAIFSGLQGPFVLLFHCVLNQEVRKHL
KGVLGGRKLHLEDSATTR
ATLLTRSLNCNTTFGDGPDMLRTDLGESTASLDSIVRDEGIQKLGVSSGLV
RGSHGEPDASLMPRSCKDP
PGHDSDSDSELSLDEQSSSYASSHSSDSEDDGVGAEEKWDPARGAVHS
TPKGDAVANHVPAGWPDQSLAE
SDSEDPSGKPRLKVETKVSVELHREEQGSHRGEYPPDQESGGAARLASS
QPPEQRKGILKNKVTYPPPLT
LTEQTLKGRLREKLADCEQSPTSSRTSSLGSGGPDCAITVKSPGREPGRD
HLNGVAMNVRTGSAQADGSD
SEKP
>SEQID3_Metagen-Variante

atggcgccgccgccgccgcccgtgctgcccgtgctgctgctcctggccgccgccgccctgccggc
gatgggg
ctgcgagcggccgcctgggagccgcgcgtacccggcgggacccgcgccttcgccctccggcccggc
tgtacctac
gcggtgggcgccgcttgcacgccccgggcgccgcgggagctgctggacgtgggccgcgatgggcgg
ctggcagga
cgtcggcgcgtctcgggcgcggggcgcccgctgccgctgcaagtccgcttggtggcccgcagtgcccc
gacggcg
ctgagccgccgcctgcgggcgcgcacgcaccttcccggctgcggagcccgtgcccggctctgcggaa
ccggtgcc
cggctctgcggggcgctctgcttccccgtccccggcggctgcgcggccgcgcagcattcggcgctcgc
agctccg
accaccttacccgcctgccgctgcccgccgcgccccaggccccgctgtcccggccgtcccatctgcctg
ccgccg
ggcggctcggtccgcctgcgtctgctgtgcgccctgcggcgcgcggctggcgCcgtccgggtgggact
ggcgctg
gaggccgccaccgcggggacgccctccgcgtcgccatccccatcgccgcccctgccgccgaacttgc
ccgaagcc
cgggcggggccggcgcgacgggcccggcggggcacgagcggcagagggagcctgaagtttccga
tgcccaactac
caggtggcgttgtttgagaacgaaccggcgggcaccctcatcctccagctgcacgcgcactacaccat
cgagggc

gaggaggagcgcgtgagctattacatggaggggctgttcgacgagcgctcccggggctacttccgaat
cgactct
gccacgggcgccgtgagcacggacagcgtactggaccgcgagaccaaggagacgcacgtcctcag
ggtgaaagcc
gtggactacagtacgccgccgcgctcggccaccacctacatcactgtcttggtcaaagacaccaacga
ccacagc
ccggtcttcgagcagtcggagtaccgcgagcgcgtgcgggagaacctggaggtgggctacgaggtgc
tgaccatc
cgcgccagcgaccgcgactcgcccatcaacgccaacttgcgttaccgcgtgttgggggggcgcgtggg
acgtcttc
cagctcaacgagagctctggcgtggtgagcacacgggcggtgctggaccgggaggaggcggccga
gtaccagctc
ctggtggaggccaacgaccaggggcgcaatccgggcccgctcagtgccacggccaccgtgtacatc
gaggtggag
gacgagaacgacaactacccccagttcagcgagcagaactacgtggtccaggtgcccgaggacgtg
gggctcaac
acggctgtgctgcgagtgcaggccacggaccgggaccagggccagaacgcggccattcactacagc
atcctcagc
gggaacgtggccggccagttctacctgcactcgctgagcgggatcctggatgtgatcaaccccttggatt
tcgag
gatgtccagaaatactcgctgagcattaaggcccaggatgggggccggcccccgctcatcaattcttca
ggggtg
gtgtctgtgcaggtgctggatgtcaacgacaacgagcctatctttgtgagcagccccttccaggccacgg

tgctg

gagaatgtgcccctgggctaccccgtggtgcacattcaggcggtggacgcggactctggagagaacg

cccggctg

cactatcgcctggtggacacggcctccacctttctgggggcggcagcgctgggcctaagaatcctgcc

cccacc

cctgacttccccttccagatccacaacagctccggttggatcacagtgtgtgccgagctggaccgcgag

gaggtg

gagcactacagcttcggggtggaggcggtggaccacggctcgccccccatgagctcctccaccagcg

tgtccatc

acggtgctggacgtgaatgacaacgacccggtgttcacgcagcccacctacgagcttcgtctgaatga

ggatgcg

gccgtggggagcagcgtgctgaccctgcaggcccgcgaccgtgacgccaacagtgtgattacctacc

agctcaca

ggcggcaacacccggaaccgctttgcactcagcagccagagagggggcggcctcatcaccctggcg

ctacctctg

gactacaagcaggagcagcagtacgtgctggcggtgacagcatccgacggcacacggtcgcacact

gcgcatgtc

ctaatcaacgtcactgatgccaacacccacaggcctgtctttcagagctcccattacacagtgagtgtca

gtgag

gacaggcctgtgggcacctccattgctaccctcagtgccaacgatgaggacacaggagagaatgccc

gcatcacc

tacgtgattcaggaccccgtgccgcagttccgcattgaccccgacagtggcaccatgtacaccatgatg

gagctg

gactatgagaaccaggtcgcctacacgctgaccatcatggcccaggacaacggcatcccgcagaaat
cagacacc
accaccctagagatcctcatcctcgatgccaatgacaatgcaccccagttcctgtgggatttctaccagg
gttcc
atctttgaggatgctccaccctcgaccagcatcctccaggtctctgccacggaccgggactcaggtccca
atggg
cgtctgctgtacaccttccagggtggggacgacggcgatggggacttctacatcgagcccacgtccggt
gtgatt
cgcacccagcgccggctggaccgggagaatgtggccgtgtacaacctttgggctctggctgtggatcg
gggcagt
cccactccccttagcgcctcggtagaaatccaggtgaccatcttggacattaatgacaatgcccccatgtt
tgag
aaggacgaactggagctgtttgttgaggagaacaacccagtggggtcggtggtggcaaagattcgtgc
taacgac
cctgatgaaggccctaatgcccagatcatgtatcagattgtggaaggggacatgcggcatttcttccagct
ggac
ctgctcaacggggacctgcgtgccatggtggagctggactttgaggtccggcgggagtatgtgctggtg
gtgcag
gccacgtcggctccgctggtgagccgagccacggtgcacatccttctcgtggaccagaatgacaaccc
gcctgtg
ctgcccgacttccagatcctcttcaacaactatgtcaccaacaagtccaacagtttccccaccggcgtgat
cggc

tgcatcccggcccatgaccccgacgtgtcagacagcctcaactacaccttcgtgcagggcaacgagct
gcgcctg
ttgctgctggaccccgccacgggcgaactgcagctcagccgcgacctggacaacaaccggccgctg
gaggcgctc
atggaggtgtctgtgtctgatggcatccacagcgtcacggccttctgcaccctgcgtgtcaccatcatcacg
gac
gacatgctgaccaacagcatcactgtccgcctggagaacatgtcccaggagaagttcctgtccccgctg
ctggcc
ctcttcgtggagggggtggccgccgtgctgtccaccaccaaggacgacgtcttcgtcttcaacgtccaga
acgac
accgacgtcagctccaacatcctgaacgtgaccttctcggcgctgctgcctggcggcgtccgcggccag
ttcttc
ccgtcggaggacctgcaggagcagatctacctgaatcggacgctgctgaccaccatctccacgcagc
gcgtgctg
cccttcgacgacaacatctgcctgcgcgagccctgcgagaactacatgaagtgcgtgtccgttctgcgat
tcgac
agctccgcgcccttcctcagctccaccaccgtgctcttccggcccatccaccccatcaacggcctgcgct
gccgc
tgcccgcccggcttcaccggcgactactgcgagacggagatcgacctctgctactccgacccgtgcgg
cgccaac
.ggccgctgccgcagccgcgagggcggctacacctgcgagtgcttcgaggacttcactggagagcact
gtgaggtg

gatgcccgctcaggccgctgtgccaacggggtgtgcaagaacgggggcacctgcgtgaacctgctca
tcggcggc
ttccactgcgtgtgtcctcctggcgagtatgagaggccctactgtgaggtgaccaccaggagcttcccgc
cccag
tccttcgtcaccttccggggcctgagacagcgcttccacttcaccatctccctcacgtttgccactcaggaa
agg
aacggcttgcttctctacaacggccgcttcaatgagaagcacgacttcatcgccctggagatcgtggac
gagcag
gtgcagctcaccttctctgcaggcgagacaacaacgaccgtggcaccgaaggttcccagtggtgtgag
tgacggg
cggtggcactctgtgcaggtgcagtactacaacaagcccaatattggccacctgggcctgccccatggg
ccgtcc
ggggaaaagatggccgtggtgacagtggatgattgtgacacaaccatggctgtgcgctttggaaagga
catcggg
aactacagctgcgctgcccagggcactcagaccggctccaagaagtccctggatctgaccggccctct
actcctg
ggggggtgtccccaacctgccagaagacttcccagtgcacaaccggcagttcgtgggctgcatgcgga
acctgtca
gtcgacggcaaaaatgtggacatggccggattcatcgccaacaatggcacccgggaaggctgcgctg
ctcggagg
aacttctgcgatgggaggcggtgtcagaatggaggcacctgtgtcaacaggtggaatatgtatctgtgtg
agtgt
ccactccgattcggcgggaagaactgtgagcaagccatgcctcacccccagctcttcagcggtgagag

cgtcgtg
tcctggagtgacctgaacatcatcatctctgtgccctggtacctggggctcatgttccggacccggaagg
aggac
agcgttctgatggaggccaccagtggtgggcccaccagctttcgcctccagatcctgaacaactacctc
cagttt
gaggtgtcccacggcccctccgatgtggagtccgtgatgctgtccgggttgcgggtgaccgacgggga
gtggcac
cacctgctgatcgagctgaagaatgttaaggaggacagtgagatgaagcacctggtcaccatgacctt
ggactat
gggatggaccagaacaaggcagatatcgggggcatgcttcccgggctgacggtaaggagcgtggtg
gtcggaggc
gcctctgaagacaaggtctccgtgcgccgtggattccgaggctgcatgcagggagtgaggatggggg
ggacgccc
accaacgtcgccaccctgaacatgaacaacgcactcaaggtcagggtgaaggacggctgtgatgtgg
acgacccc
tgtacctcgagcccctgtcccccaatagccgctgccacgacgcctgggaggactacagctgcgtctgt
gacaaa
gggtaccttggaataaactgtgtggatgcctgtcacctgaacccctgcgagaacatgggggcctgcgtg
cgctcc
cccggctccccgcagggctacgtgtgcgagtgtgggcccagtcactacgggccgtactgtgagaacaa
actcgac
cttccgtgccccagaggctggtgggggaaccccgtctgtggaccctgccactgtgccgtcagcaaagg
ctttgat

cccgactgtaataagaccaacggccagtgccaatgcaaggagaattactacaagctcctagcccagg
acacctgt
ctgccctgcgactgcttcccccatggctcccacagccgcacttgcgacatggccaccgggcagtgtgcc
tgcaag
cccggcgtcatcggccgccagtgcaaccgctgcgacaacccgtttgccgaggtcaccacgctcggctg
tgaagtg
atctacaatggctgtcccaaagcatttgaggccggcatctggtggccacagaccaagttcgggcagcc
ggctgcg
gtgccatgccctaagggatccgttggaaatgcggtccgacactgcagcggggagaagggctggctgc
ccccagag
ctctttaactgtaccaccatctccttcgtggacctcagggccatgaatgagaagctgagccgcaatgaga
cgcag
gtggacggcgccagggccctgcagctggtgagggcgctgcgcagtgctacacagcacacgggcac
gctctttggc
aatgacgtgcgcacggcctaccagctgctgggccacgtccttcagcacgagagctggcagcagggctt
cgacctg
gcagccacgcaggacgccgactttcacgaggacgtcatccactcgggcagcgccctcctggccccag
ccaccagg
gcggcgtgggagcagatccagcggagcgagggcggcacggcacagctgctccggcgcctcgagg
gctacttcagc
aacgtggcacgcaacgtgcggcggacgtacctgcggcccttcgtcatcgtcaccgccaacatgattctt
gctgtc

gacatctttgacaagttcaactttacgggagccagggtcccgcgattcgacaccatccatgaagagttcc

ccagg

gagctggagtcctccgtctccttcccagccgacttcttcagaccacctgaagaaaagaaggccccctg

ctgagg

ccggctggccggaggaccaccccgcagaccacgcgcccggggcctggcaccgaaagggaggccc

cgatcagcagg

cggaggcgacaccctgatgacgctggccagttcgccgtcgctctggtcatcatttaccgcaccctgggg

cagctc

ctgcccgagcgctacgaccccgaccgtcgcagcctccggttgcctcaccgccccatcattaataccccg

atggtg

agcacgctggtgtacagcgagggggctccgctcccgagacccctggagaggcccgtcctggtggagt

tcgccctg

ctggaggtggaggagcgaaccaagcctgtctgcgtgttctggaaccactccctggccgttggtgggacg

ggaggg

tggtctgcccggggctgcgagctcctgtccaggaaccggacacatgtcgcctgccagtgcagccacac

agccagc

tttgcggtgctcatggatatctccaggcgtgagaacggggaggtcctgcctctgaagattgtcacctatgc

cgct

gtgtccttgtcactggcagccctgctggtggccttcgtcctcctgagcctggtccgcatgctgcgctccaac

ctg

cacagcattcacaagcacctcgccgtggcgctcttcctctctcagctggtgttcgtgattgggatcaacca

gacg

gaaaacccgtttctgtgcacagtggttgccatcctcctccactacatctacatgagcacctttgcctggacc

ctc

gtggagagcctgcatgtctaccgcatgctgaccgaggtgcgcaacatcgacacggggcccatgcggtt

ctactac

gtcgtgggctggggcatcccggccattgtcacaggactggcggtcggcctggacccccagggctacgg

gaacccc

gacttctgctggctgtcgcttcaagacaccctgatttggagctttgcggggcccatcggagctgttataatca

tc

aacacagtcacttctgtcctatctgcaaaggtttcctgccaaagaaagcaccattattatgggaaaaaag

ggatc

gtctccctgctgaggaccgcattcctcctgctgctgctcatcagcgccacctggctgctggggctgctggct

gtg

aaccgcgatgcactgagctttcactacctcttcgccatcttcagcggcttacagggccccttcgtcctcctttt

c

cactgcgtgctcaaccaggaggtccggaagcacctgaagggcgtgctcggcgggaggaagctgcac

ctggaggac

tccgccaccaccagggccaccctgctgacgcgctccctcaactgcaacaccaccttcggtgacgggcc

tgacatg

ctgcgcacagacttgggcgagtccaccgcctcgctggacagcatcgtcagggatgaagggatccaga

agctcggc

gtgtcctctgggctggtgaggggcagccacggagagccagacgcgtccctcatgcccaggagctgca

aggatccc

cctggccacgattccgactcagatagcgagctgtccctggatgagcagagcagCtcttacgcctcctca

cactcg

tcagacagcgaggacgatggggtgggagctgaggaaaaatgggacccggccaggggcgccgtcc

acagcaccccc

aaaggggacgctgtggccaaccacgttccggccggctggcccgaccagagcctggctgagagtgac

agtgaggac

cccagcggcaagccccgcctgaaggtggagaccaaggtcagcgtggagctgcaccgcgaggagc

agggcagtcac

cgtggagagtacccccccggaccaggagagcgggggcgcagccaggcttgctagcagccagccccc

agagcagagg

aaaggcatcttgaaa'aataaagtcacctacccgccgccgctgacgctgacggagcagacgctgaag

ggccggctc

cgggagaagctggccgactgtgagcagagccccacatcctcgcgcacgtcttccctgggctctggcgg

ccccgac

tgcgccatcacagtcaagagccctgggagggagccggggcgtgaccacctcaacggggtggccatg

aatgtgcgc actgggagcgcccaggccgatggctccgactctgaaggcagtaatgaaacttcaatt

>SEQ-ID4_Metagen-Variante

MAPPPPPVLPVLLLLAAAAALPAMGLRAAAWEPRVPGGTRAFALRPGCTY

AVGAACTPRAPRELLDVGRDGRLAG

RRRVSGAGRPLPLQVRLVARSAPTALSRRLRARTHLPGCGARARLCGTG

ARLCGALCFPVPGGCAAAQHSALAAP

TTLPACRCPPRPRPRCPGRPICLPPGGSVRLRLLCALR.RAAGAVRVGLA

LEAATAGTPSASPSPSPPLPPNLPEA

RAGPARRARRGTSGRGSLKFPMPNYQVALFENEPAGTLILQLHAHYTIEG
EEERVSYYMEGLFDERSRGYFRIDS
ATGAVSTDSVLDRETKETHVLRVKAVDYSTPPRSATTYITVLVKDTNDHSP
VFEQSEYRERVRENLEVGYEVLTI
RASDRDSPINANLRYRVLGGAWDVFQLNESSGVVSTRAVLDREEAAEYQ
LLVEANDQGRNPGPLSATATVYIEVE
DENDNYPQFSEQNYVVQVPEDVGLNTAVLRVQATDRDQGQNAAIHYSIL
SGNVAGQFYLHSLSGILDVINPLDFE
DVQKYSLSIKAQDGGRPPLINSSGVVSVQVLDVNDNEPIFVSSPFQATVLE
NVPLGYPVVHIQAVDADSGENARL
HYRLVDTASTFLGGGSAGPKNPAPTPDFPFQIHNSSGWITVCAELDREEV
EHYSFGVEAVDHGSPPMSSSTSVSI
TVLDVNDNDPVFTQPTYELRLNEDAAVGSSVLTLQARDRDANSVITYQLT
GGNTRNRFALSSQRGGGLITLALPL
DYKQEQQYVLAVTASDGTRSHTAHVLINVTDANTHRPVFQSSHYTVSVSE
DRPVGTSIATLSANDEDTGENARIT
YVIQDPVPQFRIDPDSGTMYTMMELDYENQVAYTLTIMAQDNGIPQKSDT
TTLEILILDANDNAPQFLWDFYQGS
IFEDAPPSTSILQVSATDRDSGPNGRLLYTFQGGDDGDGDFYIEPTSGVIR
TQRRLDRENVAVYNLWALAVDRGS
PTPLSASVEIQVTILDINDNAPMFEKDELELFVEENNPVGSVVAKIRANDPD
EGPNAQIMYQIVEGDMRHFFQLD
LLNGDLRAMVELDFEVRREYVLVVQATSAPLVSRATVHILLVDQNDNPPV

LPDFQILFNNYVTNKSNSFPTGVIG
CIPAHDPDVSDSLNYTFVQGNELRLLLLDPATGELQLSRDLDNNRPLEAL
MEVSVSDGIHSVTAFCTLRVTIITD
DMLTNSITVRLENMSQEKFLSPLLALFVEGVAAVLSTTKDDVFVFNVQNDT
DVSSNILNVTFSALLPGGVRGQFF
PSEDLQEQIYLNRTLLTTISTQRVLPFDDNICLREPCENYMKCVSVLRFDS
SAPFLSSTTVLFRPIHPINGLRCR
CPPGFTGDYCETEIDLCYSDPCGANGRCRSREGGYTCECFEDFTGEHCE
VDARSGRCANGVCKNGGTCVNLLIGG
FHCVCPPGEYERPYCEVTTRSFPPQSFVTFRGLRQiWHFTISLTFATQER
NGLLLYNGRFNEKHDFIALEIVDEQ
VQLTFSAGETTTTVAPKVPSGVSDGRWHSVQVQYYNKPNIGHLGLPHGP
SGEKMAVVTVDDCDTTMAVRFGKDIG
NYSCAAQGTQTGSKKSLDLTGPLLLGGVPNLPEDFPVHNRQFVGCMRNL
SVDGKNVDMAGFIANNGTREGCAARR
NFCDGRRCQNGGTCVNRWNMYLCECPLRFGGKNCEQAMPHPQLFSGE
SVVSWSDLNIIISVPWYLGLMFRTRKED
SVLMEATSGGPTSFRLQILNNYLQFEVSHGPSDVESVMLSGLRVTDGEW
HHLLIELKNVKEDSEMKHLVTMTLDY
GMDQNKADIGGMLPGLTVRSVVVGGASEDKVSVRRGFRGCMQGVRMG
GTPTNVATLNMNNALKVRVKDGCDVDDP
CTSSPCPPNSRCHDAWEDYSCVCDKGYLGINCVDACHLNPCENMGACV
RSPGSPQGYVCECGPSHYGPYCENKLD

LPCPRGWWGNPVCGPCHCAVSKGFDPDCNKTNGQCQCKENYYKLLAQ

DTCLPCDCFPHGSHSRTCDMATGQCACK

PGVIGRQCNRCDNPFAEVTTLGCEVIYNGCPKAFEAGIWWPQTKFGQPA

AVPCPKGSVGNAVRHCSGEKGWLPPE

LFNCTTISFVDLRAMNEKLSRNETQVDGARALQLVRALRSATQHTGTLFG

NDVRTAYQLLGHVLQHESWQQGFDL

AATQDADFHEDVIHSGSALLAPATRAAWEQIQRSEGGTAQLLRRLEGYFS

NVARNV:RRTYLRPFVIVTANMILAV

DIFDKFNFTGARVPRFDTIHEEFPRELESSVSFPADFFRPPEEKEGPLLRP

AGRRTTPQTTRPGPGTEREAPISR

RRRHPDDAGQFAVALVIIYRTLGQLLPERYDPDRRSLRLPHRPIINTPMVS

TLVYSEGAPLPRPLERPVLVEFAL

LEVEERTKPVCVFWNHSLAVGGTGGWSARGCELLSRNRTHVACQCSHT

ASFAVLMDISRRENGEVLPLKIVTYAA

VSLSLAALLVAFVLLSLVRMLRSNLHSIHKHLAVALFLSQLVFVIGINQTENP

FLCTVVAILLHYIYMSTFAWTL

VESLHVYRMLTEVRNIDTGPMRFYYVVGWGIPAIVTGLAVGLDPQGYGNP

DFCWLSLQDTLIWSFAGPIGAVIII

NTVTSVLSAKVSCQRKHHYYGKKGIVSLLRTAFLLLLLISATWLLGLLAVNR

DALSFHYLFAIFSGLQGPFVLLF

HCVLNQEVRKHLKGVLGGRKLHLEDSATTRATLLTRSLNCNTTFGDGPD

MLRTDLGESTASLDSIVRDEGIQKLG

VSSGLVRGSHGEPDASLMPRSCKDPPGHDSDSDSELSLDEQSSSYASS

HSSDSEDDGVGAEEKWDPARGAVHSTP

KGDAVANHVPAGWPDQSLAESDSEDPSGKPRLKVETKVSVELHREEQG

SHRGEYPPDQESGGAARLASSQPPEQ^R

KGILKNKVTYPPPLTLTEQTLKGRLREKLADCEQSPTSSRTSSLGSGGPD

CAITVKSPGREPGRDHLNGVAMNVR TGSAQADGSDSEGSNETSI

>SEQID5_N-terminal_extrazellulär

ATGGCGCCGCCGCCGCCCGTGCTGCCCGTGCTGCTGCTCCTGG

CCGCCGCCGCCGCCCTGCCGGCGA

TGGGGCTGCGAGCGGCCGCCTGGGAGCCGCGCGTACCCGGCGGAC

CCGCGCCTTCGCCCTCCGGCCCGG

CTGTACCTACGCGGTGGGCGCCGCTTGCACGCCCCGGGCGCCGCGG

GAGCTGCTGGACGTGGGCCGCGAT

GGGCGGCTGGCAGGACGTCGGCGCGTCTCGGGCGCGGGCGCCCG

CTGCCGCTGCAAGTCCGCTTGGTGG

CCCGCAGTGCCCCGACGGCGCTGAGCCGCCGCCTGCGGGCGCGCAC

GCACCTTCCCGGCTGCGGAGCCCG

TGCCCGGCTCTGCGGAACCGGTGCCCGGCTCTGCGGGGCGCTCTGC

TTCCCCGTCCCCGGCGGCTGCGCG

GCCGCGCAGCATTCGGCGCTCGCAGCTCCGACCACCTTACCCGCCTG

CCGCTGCCCGCCGCGCCCCAGGC

CCCGCTGTCCCGGCCGTCCCATCTGCCTGCCGCCGGGCGGCTCGGT
CCGCCTGCGTCTGCTGTGCGCCCT
GCGGCGCGCGGCTGGCGCCGTCCGGGTGGGACTGGCGCTGGAGGC
CGCCACCGCGGGGACGCCCTCCGCG
TCGCCATCCCCATCGCCGCCCCTGCCGCCGAACTTGCCCGAAGCCCG
GGCGGGGCCGGCGCGACGGGCCC
GGCGGGGCACGAGCGGCAGAGGGAGCCTGAAGTTTCCGATGCCCAA
CTACCAGGTGGCGTTGTTTGAGAA
CGAACCGGCGGGCACCCTCATCCTCCAGCTGCACGCGCACTACACCA
TCGAGGGCGAGGAGGAGCGCGTG
AGCTATTACATGGAGGGGCTGTTCGACGAGCGCTCCCGGGGCTACTT
CCGAATCGACTCTGCCACGGGCG
CCGTGAGCACGGACAGCGTACTGGACCGCGAGACCAAGGAGACGCAC
GTCCTCAGGGTGAAAGCCGTGGA
CTACAGTACGCCGCCGCGCTCGGCCACCACCTACATCACTGTCTTGGT
CAAAGACACCAACGACCACAGC
CCGGTCTTCGAGCAGTCGGAGTACCGCGAGCGCGTGCGGGAGAACCT
GGAGGTGGGCTACGAGGTGCTGA
CCATCCGCGCCAGCGACCGCGACTCGCCCATCAACGCCAACTTGCGT
TACCGCGTGTTGGGGGGCGCGTG

GGACGTCTTCCAGCTCAACGAGAGCTCTGGCGTGGTGAGCACACGGG
CGGTGCTGGACCGGGAGGAGGCG
GCCGAGTACCAGCTCCTGGTGGAGGCCAACGACCAGGGGCGCAATCC
GGGCCCGCTCAGTGCCACGGCCA
CCGTGTACATCGAGGTGGAGGACGAGAACGACAACTACCCCCAGTTC
AGCGAGCAGAACTACGTGGTCCA
GGTGCCCGAGGACGTGGGGCTCAACACGGCTGTGCTGCGAGTGCAG
GCCACGGACCGGGACCAGGGCCAG
AACGCGGCCATTCACTACAGCATCCTCAGCGGGAACGTGGCCGGCCA
GTTCTACCTGCACTCGCTGAGCG
GGATCCTGGATGTGATCAACCCCTTGGATTTCGAGGATGTCCAGAAAT
ACTCGCTGAGCATTAAGGCCCA
GGATGGGGGCCGGCCCCCGCTCATCAATTCTTCAGGGGTGGTGTCTG
TGCAGGTGCTGGATGTCAACGAC
AACGAGCCTATCTTTGTGAGCAGCCCCTTCCAGGCCACGGTGCTGGA
GAATGTGCCCCTGGGCTACCCCG
TGGTGCACATTCAGGCGGTGGACGCGGACTCTGGAGAGAACGCCCGG
CTGCACTATCGCCTGGTGGACAC
GGCCTCCACCTTTCTGGGGGGCGGCAGCGCTGGGCCTAAGAATCCTG
CCCCCACCCCTGACTTCCCCTTC

CAGATCCACAACAGCTCCGGTTGGATCACAGTGTGTGCCGAGCTGGA

CCGCGAGGAGGTGGAGCACTACA

GCTTCGGGGTGGAGGCGGTGGACCACGGCTCGCCCCCATGAGCTC

CTCCACCAGCGTGTCCATCACGGT

GCTGGACGTGAATGACAACGACCCGGTGTTCACGCAGCCCACCTACG

AGCTTCGTCTGAATGAGGATGCG

GCCGTGGGGAGCAGCGTGCTGACCCTGCAGGCCCGCGACCGTGACG

CCAACAGTGTGATTACCTACCAGC

TCACAGGCGGCAACACCCGGAACCGCTTTGCACTCAGCAGCCAGAGA

GGGGGCGGCCTCATCACCCTGGC

GCTACCTCTGGACTACAAGCAGGAGCAGCAGTACGTGCTGGCGGTGA

CAGCATCCGACGGCACACGGTCG

CACACTGCGCATGTCCTAATCAACGTCACTGATGCCAACACCCACAGG

CCTGTCTTTCAGAGCTCCCATT

ACACAGTGAGTGTCAGTGAGGACAGGCCTGTGGGCACCTCCATTGCTA

CCCTCAGTGCCAACGATGAGGA

CACAGGAGAGAATGCCCGCATCACCTACGTGATTCAGGACCCCGTGC

CGCAGTTCCGCATTGACCCCGAC

AGTGGCACCATGTACACCATGATGGAGCTGGACTATGAGAACCAGGTC

GCCTACACGCTGACCATCATGG

CCCAGGACAACGGCATCCCGCAGAAATCAGACACCACCACCCTAGAG
ATCCTCATCCTCGATGCCAATGA
CAATGCACCCCAGTTCCTGTGGGATTTCTACCAGGGTTCCATCTTTGA
GGATGCTCCACCCTCGACCAGC
ATCCTCCAGGTCTCTGCCACGGACCGGGACTCAGGTCCCAATGGGCG
TCTGCTGTACACCTTCCAGGGTG
GGGACGACGGCGATGGGGACTTCTACATCGAGCCCACGTCCGGTGTG
ATTCGCACCCAGCGCCGGCTGGA
CCGGGAGAATGTGGCCGTGTACAACCTTTGGGCTCTGGCTGTGGATC
GGGGCAGTCCCACTCCCCTTAGC
GCCTCGGTAGAAATCCAGGTGACCATCTTGGACATTAATGACAATGCC
CCCATGTTTGAGAAGGACGAAC
TGGAGCTGTTTGTTGAGGAGAACAACCCAGTGGGGTCGGTGGTGGCA
AAGATTCGTGCTAACGACCCTGA
TGAAGGCCCTAATGCCCAGATCATGTATCAGATTGTGGAAGGGGACAT
GCGGCATTTCTTCCAGCTGGAC
CTGCTCAACGGGGACCTGCGTGCCATGGTGGAGCTGGACTTTGAGGT
CCGGCGGGAGTATGTGCTGGTGG
TGCAGGCCACGTCGGCTCCGCTGGTGAGCCGAGCCACGGTGCACATC
CTTCTCGTGGACCAGAATGACAA

CCCGCCTGTGCTGCCCGACTTCCAGATCCTCTTCAACAACTATGTCAC
CAACAAGTCCAACAGTTTCCCC
ACCGGCGTGATCGGCTGCATCCCGGCCCATGACCCCGACGTGTCAGA
CAGCCTCAACTACACCTTCGTGC
AGGGCAACGAGCTGCGCCTGTTGCTGCTGGACCCCGCCACGGGCGAA
CTGCAGCTCAGCCGCGACCTGGA
CAACAACCGGCCGCTGGAGGCGCTCATGGAGGTGTCTGTGTCTGATG
GCATCCACAGCGTCACGGCCTTC
TGCACCCTGCGTGTCACCATCATCACGGACGACATGCTGACCAACAGC
ATCACTGTCCGCCTGGAGAACA
TGTCCCAGGAGAAGTTCCTGTCCCCGCTGCTGGCCCTCTTCGTGGAG
GGGGTGGCCGCCGTGCTGTCCAC
CACCAAGGACGACGTCTTCGTCTTCAACGTCCAGAACGACACCGACGT
CAGCTCCAACATCCTGAACGTG
ACCTTCTCGGCGCTGCTGCCTGGCGGCGTCCGCGGCCAGTTCTTCCC
GTCGGAGGACCTGCAGGAGCAGA
TCTACCTGAATCGGACGCTGCTGACCACCATCTCCACGCAGCGCGTGC
TGCCCTTCGACGACAACATCTG
CCTGCGCGAGCCCTGCGAGAACTACATGAAGTGCGTGTCCGTTCTGC
GATTCGACAGCTCCGCGCCCTTC

CTCAGCTCCACCACCGTGCTCTTCCGGCCCATCCACCCCATCAACGGC
CTGCGCTGCCGCTGCCCGCCCG
GCTTCACCGGCGACTACTGCGAGACGGAGATCGACCTCTGCTACTCC
GACCCGTGCGGCGCCAACGGCCG
CTGCCGCAGCCGCGAGGGCGGCTACACCTGCGAGTGCTTCGAGGACT
TCACTGGAGAGCACTGTGAGGTG
GATGCCCGCTCAGGCCGCTGTGCCAACGGGGTGTGCAAGAACGGGG
GCACCTGCGTGAACCTGCTCATCG
GCGGCTTCCACTGCGTGTGTCCTCCTGGCGAGTATGAGAGGCCCTAC
TGTGAGGTGACCACCAGGAGCTT
CCCGCCCCAGTCCTTCGTCACCTTCCGGGGCCTGAGACAGCGCTTCC
ACTTCACCATCTCCCTCACGTTT
GCCACTCAGGAAAGGAACGGCTTGCTTCTCTACAACGGCCGCTTCAAT
GAGAAGCACGACTTCATCGCCC
TGGAGATCGTGGACGAGCAGGTGCAGCTCACCTTCTCTGCAGGCGAG
ACAACAACGACCGTGGCACCGAA
GGTTCCCAGTGGTGTGAGTGACGGGCGGTGGCACTCTGTGCAGGTGC
AGTACTACAACAAGCCCAATATT
GGCCACCTGGGCCTGCCCCATGGGCCGTCCGGGGAAAAGATGGCCG
TGGTGACAGTGGATGATTGTGACA

CAACCATGGCTGTGCGCTTTGGAAAGGACATCGGGAACTACAGCTGC
GCTGCCCAGGGCACTCAGACCGG
CTCCAAGAAGTCCCTGGATCTGACCGGCCCTCTACTCCTGGGGGGTG
TCCCCAACCTGCCAGAAGACTTC
CCAGTGCACAACCGGCAGTTCGTGGGCTGCATGCGGAACCTGTCAGT
CGACGGCAAAAATGTGGACATGG
CCGGATTCATCGCCAACAATGGCACCCGGGAAGGCTGCGCTGCTCGG
AGGAACTTCTGCGATGGGAGGCG
GTGTCAGAATGGAGGCACCTGTGTCAACAGGTGGAATATGTATCTGTG
TGAGTGTCCACTCCGATTCGGC
GGGAAGAACTGTGAGCAAGCCATGCCTCACCCCCAGCTCTTCAGCGG
TGAGAGCGTCGTGTCCTGGAGTG
ACCTGAACATCATCATCTCTGTGCCCTGGTACCTGGGGCTCATGTTCC
GGACCCGGAAGGAGGACAGCGT
TCTGATGGAGGCCACCAGTGGTGGGCCCACCAGCTTTCGCCTCCAGA
TCCTGAACAACTACCTCCAGTTT
GAGGTGTCCCACGGCCCCTCCGATGTGGAGTCCGTGATGCTGTCCGG
GTTGCGGGTGACCGACGGGGAGT
GGCACCACCTGCTGATCGAGCTGAAGAATGTTAAGGAGGACAGTGAG
ATGAAGCACCTGGTCACCATGAC

CTTGGACTATGGGATGGACCAGAACAAGGCAGATATCGGGGGCATGC
TTCCCGGGCTGACGGTAAGGAGC
GTGGTGGTCGGAGGCGCCTCTGAAGACAAGGTCTCCGTGCGCCGTGG
ATTCCGAGGCTGCATGCAGGGAG
TGAGGATGGGGGGGACGCCCACCAACGTCGCCACCCTGAACATGAAC
AACGCACTCAAGGTCAGGGTGAA
GGACGGCTGTGATGTGGACGACCCCTGTACCTCGAGCCCCTGTCCCC
CCAATAGCCGCTGCCACGACGCC
TGGGAGGACTACAGCTGCGTCTGTGACAAAGGGTACCTTGGAATAAAC
TGTGTGGATGCCTGTCACCTGA
ACCCCTGCGAGAACATGGGGGCCTGCGTGCGCTCCCCCGGCTCCCC
GCAGGGCTACGTGTGCGAGTGTGG
GCCCAGTCACTACGGGCCGTACTGTGAGAACAAACTCGACCTTCCGTG
CCCCAGAGGCTGGTGGGGGAAC
CCCGTCTGTGGACCCTGCCACTGTGCCGTCAGCAAAGGCTTTGATCCC
GACTGTAATAAGACCAACGGCC
AGTGCCAATGCAAGGAGAATTACTACAAGCTCCTAGCCCAGGACACCT
GTCTGCCCTGCGACTGCTTCCC
CCATGGCTCCCACAGCCGCACTTGCGACATGGCCACCGGGCAGTGTG
CCTGCAAGCCCGGCGTCATCGGC

CGCCAGTGCAACCGCTGCGACAACCCGTTTGCCGAGGTCACCACGCT
CGGCTGTGAAGTGATCTACAATG
GCTGTCCCAAAGCATTTGAGGCCGGCATCTGGTGGCCACAGACCAAG
TTCGGGCAGCCGGCTGCGGTGCC
ATGCCCTAAGGGATCCGTTGGAAATGCGGTCCGACACTGCAGCGGGG
AGAAGGGCTGGCTGCCCCCAGAG
CTCTTTAACTGTACCACCATCTCCTTCGTGGACCTCAGGGCCATGAATG
AGAAGCTGAGCCGCAATGAGA
CGCAGGTGGACGGCGCCAGGGCCCTGCAGCTGGTGAGGGCGCTGCG
CAGTGCTACACAGCACACGGGCAC
GCTCTTTGGCAATGACGTGCGCACGGCCTACCAGCTGCTGGGCCACG
TCCTTCAGCACGAGAGCTGGCAG
CAGGGCTTCGACCTGGCAGCCACGCAGGACGCCGACTTTCACGAGGA
CGTCATCCACTCGGGCAGCGCCC
TCCTGGCCCCAGCCACCAGGGCGGCGTGGGAGCAGATCCAGCGGAG
CGAGGGCGGCACGGCACAGCTGCT
CCGGCGCCTCGAGGGCTACTTCAGCAACGTGGCACGCAACGTGCGGC
GGACGTACCTGCGGCCCTTCGTC
ATCGTCACCGCCAACATGATTCTTGCTGTCGACATCTTTGACAAGTTCA
ACTTTACGGGAGCCAGGGTCC

CGCGATTCGACACCATCCATGAAGAGTTCCCCAGGGAGCTGGAGTCCT
CCGTCTCCTTCCCAGCCGACTT
CTTCAGACCACCTGAAGAAAAGAAGGCCCCCTGCTGAGGCCGGCTG
GCCGGAGGACCACCCCGCAGACC
ACGCGCCCGGGGCCTGGCACCGAGAGGGAGGCCCCGATCAGCAGGC
GGAGGCGACACCCTGATGACGCTG
GCCAGTTCGCCGTCGCTCTGGTCATCATTTACCGCACCCTGGGGCAG
CTCCTGCCCGAGCGCTACGACCC
CGACCGTCGCAGCCTCCGGTTGCCTCACCGGCCCATCATTAATACCCC
GATGGTGAGCACGCTGGTGTAC
AGCGAGGGGGCTCCGCTCCCGAGACCCCTGGAGAGGCCCGTCCTGG
TGGAGTTCGCCCTGCTGGAGGTGG
AGGAGCGAACCAAGCCTGTCTGCGTGTTCTGGAACCACTCCCTGGCC
GTTGGTGGGACGGGAGGGTGGTC
TGCCCGGGGCTGCGAGCTCCTGTCCAGGAACCGGACACATGTCGCCT
GCCAGTGCAGCCACACAGCCAGC
TTTGCGGTGCTCATGGATATCTCCAGGCGTGAGAACGGGGAGGTCCT
GCCTCTGAA
>SEQID6_N-terminal_extrazellulär
MAPPPPPVLPVLLLLAAAAALPAMGLRAAAWEPRVPGGTRAFALRPGCTY

AVGAACTPRAPRELLDVGRD

GRLAGRRRVSGAGRPLPLQVRLVARSAPTALSRRLRARTHLPGCGARAR

LCGTGARLCGALCFPVPGGCA

AAQHSALAAPTTLPACRCPPRPRPRCPGRPICLPPGGSVRLRLLCALRRA

AGAVRVGLALEAATAGTPSA

SPSPSPPLPPNLPEARAGPARRARRGTSGRGSLKFPMPNYQVALFENEP

AGTLILQLHAHYTIEGEEERV

SYYMEGLFDERSRGYFRIDSATGAVSTDSVLDRETKETHVLRVKAVDYST

PPRSATTYITVLVKDTNDHS

PVFEQSEYRERVRENLEVGYEVLTIRASDRDSPINANLRYRVLGGAWDVF

QLNESSGVVSTRAVLDREEA

AEYQLLVEANDQGRNPGPLSATATVYIEVEDENDNYPQFSEQNYVVQVPE

DVGLNTAVLRVQATDRDQGQ

NAAIHYSILSGNVAGQFYLHSLSGILDVINPLDFEDVQKYSLSIKAQDGGRP

PLINSSGVVSVQVLDVND

NEPIFVSSPFQATVLENVPLGYPVVHIQAVDADSGENARLHYRLVDTASTF

LGGGSAGPKNPAPTPDFPF

QIHNSSGWITVCAELDREEVEHYSFGVEAVDHGSPPMSSSTSVSITVLDV

NDNDPVFTQPTYELRLNEDA

AVGSSVLTLQARDRDANSVITYQLTGGNTRNRFALSSQRGGGLITLALPLD

YKQEQQYVLAVTASDGTRS

HTAHVLINVTDANTHRPVFQSSHYTVSVSEDRPVGTSIATLSANDEDTGEN

ARITYVIQDPVPQFRIDPD

SGTMYTMMELDYENQVAYTLTIMAQDNGIPQKSDTTTLEILILDANDNAPQ

FLWDFYQGSIFEDAPPSTS

ILQVSATDRDSGPNGRLLYTFQGGDDGDGDFYIEPTSGVIRTQRRLDREN

VAVYNLWALAVDRGSPTPLS

ASVEIQVTILDINDNAPMFEKDELELFVEENNPVGSVVAKIRANDPDEGPNA

QIMYQIVEGDMRHFFQLD

LLNGDLRAMVELDFEVRREYVLVVQATSAPLVSRATVHILLVDQNDNPPVL

PDFQILFNNYVTNKSNSFP

TGVIGCIPAHDPDVSDSLNYTFVQGNELRLLLLDPATGELQLSRDLDNNRP

LEALMEVSVSDGIHSVTAF

CTLRVTIITDDMLTNSITVRLENMSQEKFLSPLLALFVEGVAAVLSTTKDDVF

VFNVQNDTDVSSNILNV

TFSALLPGGVRGQFFPSEDLQEQIYLNRTLLTTISTQRVLPFDDNICLREPC

ENYMKCVSVLRFDSSAPF

LSSTTVLFRPIHPINGLRCRCPPGFTGDYCETEIDLCYSDPCGANGRCRSR

EGGYTCECFEDFTGEHCEV

DARSGRCANGVCKNGGTCVNLLIGGFHCVCPPGEYERPYCEVTTRSFPP

QSFVTFRGLRQRFHFTISLTF

ATQERNGLLLYNGRFNEKHDFIALEIVDEQVQLTFSAGETTTTVAPKVPSG

VSDGRWHSVQVQYYNKPNI

GHLGLPHGPSGEKMAVVTVDDCDTTMAVRFGKDIGNYSCAAQGTQTGSK

KSLDLTGPLLLGGVPNLPEDF

PVHNRQFVGCMRNLSVDGKNVDMAGFIANNGTREGCAARRNFCDGRRC

QNGGTCVNRWNMYLCECPLRFG

GKNCEQAMPHPQLFSGESVVSWSDLNIIISVPWYLGLMFRTRKEDSVLME

ATSGGPTSFRLQILNNYLQF

EVSHGPSDVESVMLSGLRVTDGEWHHLLIELKNVKEDSEMKHLVTMTLDY

GMDQNKADIGGMLPGLTVRS

VVVGGASEDKVSVRRGFRGCMQGVRMGGTPTNVATLNMNNALKVRVKD

GCDVDDPCTSSPCPPNSRCHDA

WEDYSCVCDKGYLGINCVDACHLNPCENMGACVRSPGSPQGYVCECGP

SHYGPYCENKLDLPCPRGWWGN

PVCGPCHCAVSKGFDPDCNKTNGQCQCKENYYKLLAQDTCLPCDCFPH

GSHSRTCDMATGQCACKPGVIG

RQCNRCDNPFAEVTTLGCEVIYNGCPKAFEAGIWWPQTKFGQPAAVPCP

KGSVGNAVRHCSGEKGWLPPE

LFNCTTISFVDLRAMNEKLSRNETQVDGARALQLVRALRSATQHTGTLFG

NDVRTAYQLLGHVLQHESWQ

QGFDLAATQDADFHEDVIHSGSALLAPATRAAWEQIQRSEGGTAQLLRRL

EGYFSNVARNVRRTYLRPFV

IVTANMILAVDIFDKFNFTGARVPRFDTIHEEFPRELESSVSFPADFFRPPE

EKEGPLLRPAGRRTTPQT

TRPGPGTEREAPISRRRRHPDDAGQFAVALVIIYRTLGQLLPERYDPDRRS

LRLPHRPIINTPMVSTLVY

SEGAPLPRPLERPVLVEFALLEVEERTKPVCVFWNHSLAVGGTGGWSAR

GCELLSRNRTHVACQCSHTAS

FAVLMDISRRENGEVLPLK

>SEQID7_N-terminal_Fragment

GGCTTCGACCTGGCAGCCACGCAGGACGCCGACTTTCACGAGGACGT

CATCCACTCGGGCAGCGCCC

TCCTGGCCCCAGCCACCAGGGCGGCGTGGGAGCAGATCCAGCGGAG

CGAGGGCGGCACGGCACAGCTGCT

CCGGCGCCTCGAGGGCTACTTCAGCAACGTGGCACGCAACGTGCGGC

GGACGTACCTGCGGCCCTTCGTC

ATCGTCACCGCCAACATGATTCTTGCTGTCGACATCTTTGACAAGTTCA

ACTTTACGGGAGCCAGGGTCC

CGCGATTCGACACCATCCATGAAGAGTTCCCCAGGGAGCTGGAGTCCT

CCGTCTCCTTCCCAGCCGACTT

CTTCAGACCACCTGAAGAAAAAGAAGGCCCCCTGCTGAGGCCGGCTG

GCCGGAGGACCACCCCGCAGACC

ACGCGCCCGGGGCCTGGCACCGAGAGGGAGGCCCCGATCAGCAGGC

GGAGGCGACACCCTGATGACGCTG

GCCAGTTCGCCGTCGCTCTGGTCATCATTTACCGCACCCTGGGGCAG

CTCCTGCCCGAGCGCTACGACCC

CGACCGTCGCAGCCTCCGGTTGCCTCACCGGCCCATCATTAATACCCC

GATGGTGAGCACGCTGGTGTAC

AGCGAGGGGGCTCCGCTCCCGAGACCCCTGGAGAGGCCCGTCCTGG

TGGAGTTCGCCCTGCTGGAGGTGG

AGGAGCGA

SEQID8_N-terminal_Fragment

GFDLAATQDADFHEDVIHSGSALLAPATRAAWEQIQRSEGGTAQLLRRLE

GYFSNVARNVRRTYLRPFV

IVTANMILAVDIFDKFNFTGARVPRFDTIHEEFPRELESSVSFPADFFRPPE

EKEGPLLRPAGRRTTPQT

TRPGPGTEREAPISRRRRHPDDAGQFAVALVIIYRTLGQLLPERYDPDRRS

LRLPHRPIINTPMVSTLVY

SEGAPLPRPLERPVLVEFALLEVEER

SEQID9

Loop 1 (9bp)

Gaaaacccg

SEQID10

Loop1 (3AA)

ENP

SEQID11

Loop 2 (57 bp)

gacccccagggctacgggaaccccgacttctgctggctgtcgcttcaagacaccctg

SEQID12

Loop 2 (19 AA)

DPQGYGNPDFCWLSLQDTL

SEQID13

Loop 3 (27bp)

Ctggctgtgaaccgcgatgcactgagc

SEQID14

Loop 3 (9 AA)

LAVNRDALS

>SEQID15_Sonde-B

gcgctgagcgggatcctggatgtgatcaacccccttggatttcgaggatgtccagaaatactcgctgagca

ttaag

gcccaggatgggggccggcccccgctcatcaattcttcaggggtggtgtctgtgcaggtgctggatgtca

acgac

aacgagcctatctttgtgagcagccccttccaggccacggtgctggagaatgtgcccctgggctacccc

gtggtg

cacattcaggcggtggacgcggactctggagagaacgcccggctgcactatcgcctggtggacacgg

cctccacc

tttctgggggggcggcagcgctgggcctaagaatcctgcccccacccctgacttccccttcbagatccaca

acagc

tccggttggatcacagtgtgtgccgagctggaccgcgaggaggtggagcactacagcttcggggtgga

ggcggtg

gaccacggctcgccccccatgagctcctccaccagcgtgtccatcacggtgctggacgtgaatgacaa

cgacccg

gtgttcacgcagcccacctacgagcttcgtctgaatgaggatgcggccgtggggagcagcgtgctgac

cctgcag

gcccgcgaccgtgacgccaacagtgtgattacctaccagctcacaggcggcaacacccggaaccgc

tttgcactc

agcagccagagagggggcggcctcatcaccctggcgctacctctggactacaagcaggagcagca

gtacgtgctg

gcggtgacagcatccgacggcacacggtcgcacactgcgcatgtcctaatcaacgtcactgatgccaa

cacccac

aggcctgtctttcagagctcccattacacagtgagtgtcagtgaggacaggcctgtgggcacctccattg

ctacc

ctcagtgccaacgatgaggacacaggagagaatgcccgcatcacctacgtgattcaggaccccgtgc

cgcagttc

cgcattgaccccgacagtggcaccatgtacaccatgatggagctggactatgagaaccaggtcgccta

cacgctg

accatcatggcccaggacaacggcatcccgcagaaatcagacaccaccaccctagagatcctcatcc

tcgatgcc

aatgacaatgcaccccagttcctgtgggatttctaccagggttccatctttgaggatgctccaccctcgacc

agc

atcctccaggtctctgccacggaccgggactcaggtcccaatgggcgtctgctgtacaccttccagggtg

gggac

gacggcgatggggacttctacatcgagcccacgtccggtgtgattcgcacccagcgccggctggaccg

ggagaat

gtggccgtgtacaacctttgggctctggctgtggatcggggcagtcccactccccttagcgcctcggtaga

aatc

caggtgaccatcttggacattaatgacaatgcccccatgtttgagaaggacgaactggagctgtttgttga

ggag

aacaacccagtggggtcggtggtggcaaagattcgtgctaacgaccctgatgaaggccctaatgccca

gatcatg

tatcagattgtggaaggggacatgcggcatttcttccagctggacctgctcaacggggacctgcgtgcca

tggtg

gagctggactttgaggtccggcgggagtatgtgctggtggtgcaggccacgtcggctccgctggtgagc
cgagcc
acggtgcacatccttctcgtggaccagaatgacaacccgcctgtgctgcccgacttccagatcctcttca
acaac
tatgtcaccaacaagtccaacagtttccccaccggcgtgatcggctgcatcccggcccatgaccccgac
gtgtca
gacagcctcaactacaccttcgtgcagggcaacgagctgcgcctgttgctgctggaccccgccacggg
cgaactg
cagctcagccgcgacctggacaacaaccggccgctggaggcgctcatggaggtgtctgtgtctgatgg
catccac
agcgtcacggccttctgcaccctgcgtgtcaccatcatcacggacgacatgctgaccaacagcatcact
gtccgc
ctggagaacatgtcccaggagaagttcctgtccccgctgctggccctcttcgtggagggggtggccgcc
gtgctg
tccaccaccaaggacgacgtcttcgtcttcaacgtccagaacgacaccgacgtcagctccaacatcctg
aacgtg accttctcggcgctgctgcctggcggcgtccgcggccagttcttcccg
SEQID16
aa cgagccuaucuuugugagc dTdT
SEQID17
21954
ACAGCCUCAACUACACCUUCGUG

SEQID18

24578mm

ACAGCCUCUACAACACCUUGGUG

SEQID19

21955

AGGAGCAGAUCUACCUGAAUCGG

SEQID20

24579mm

AGCAGCAGAACUUCCUGAAUGGG

SEQID21

21959

CGACUUCUUCAGACCACCUGAAG

SEQID22

24580mm

CGAGUUCUUCACAGCACCUCAAG

SEQID23

21952

GCCAUUCACUACAGCAUCCUCAG

SEQID24

21956

GAGGACUUCACUGGAGAGCACUG

SEQID25

21957

GACAGUGAGAUGAAGCACCUGGU

SEQID26

21958

GAUUCGACACCAUCCAUGAAGAG

SEQID27

15187

GACCAUGGGACCUUAAAGCGUUG

SEQID28

15199

UGGACAGAGGUGUGGGCCUUCC

SEQID29

15211

GGUGUGGGGCCUUCCAGGCCGUU

SEQID30

15223

CUCCAGGCAGGGUUCUCCCCUCU

SEQID31 15235

AGAGGGACAAAGCCACAGCCACA

SEQID32

15247

AGUGAUCAGCACAGCUGCUUCCA

SEQID33

15259

AAUGGUCCAGGACAGGAGGAUGA

SEQID34

15271

CCACAGCCAACAUCACGAGGUGU

>SEQID35_C-terminus

gggcttcgacctggcagccacgcaggacgccgactttcacgaggacgtcatccactcgggcagcgcc

ctcctggc

cccagccaccagggcggcgtgggagcagatccagcggagcgagggcggcacggcacagctgctc

cggcgcctcga

gggctacttcagcaacgtggcacgcaacgtgcggcggacgtacctgcggcccttcgtcatcgtcaccg

ccaacat

gattcttgctgtcgacatctttgacaagttcaactttacgggagccagggtcccgcgattcgacaccatcc

atga

agagttccccagggagctggagtcctccgtctccttcccagccgacttcttcagaccacctgaagaaaa

agaagg

ccccctgctgaggccggctggccggaggaccaccccgcagaccacgcgcccggggcctggcaccg

agagggaggc

cccgatcagcaggcggaggcgacaccctgatgacgctggccagttcgccgtcgctctggtcatcattta

ccgcac

cctggggcagctcctgcccgagcgctacgaccccgaccgtcgcagcctccggttgcctcaccggccca

tcattaa

taccccgatggtgagcacgctggtgtacagcgagggggctccgctcccgagacccctggagaggccc

gtcctggt

ggagttcgccctgctggaggtggaggagcgaaccaagcctgtctgcgtgttctggaaccactccctggc

cgttgg

tgggacgggagggtggtctgcccggggctgcgagctcctgtccaggaaccggacacatgtcgcctgc

cagtgcag

ccacacagccagctttgcggtgctcatggatatctccaggcgtgagaacggggaggtcctgcctctgaa

gattgt

cacctatgccgctgtgtccttgtcactggcagccctgctggtggccttcgtcctcctgagcctggtccgcatg

ct

gcgctccaacctgcacagcattcacaagcacctcgccgtggcgctcttcctctctcagctggtgttcgtgat
tgg
gatcaaccagacggaaaacccgtttctgtgcacagtggttgccatcctcctccactacatctacatgagc
acctt
tgcctggaccctcgtggagagcctgcatgtctaccgcatgctgaccgaggtgcgcaacatcgacacgg
ggcccat
gcggttctactacgtcgtgggctggggcatcccggccattgtcacaggactggcggtcggcctggaccc
ccaggg
ctacgggaaccccgacttctgctggctgtcgcttcaagacaccctgatttggagctttgcggggcccatcg
gagc
tgttataatcatcaacacagtcacttctgtcctatctgcaaaggtttcctgccaaagaaagcaccattattat
gg
gaaaaaagggatcgtctccctgctgaggaccgcattcctcctgctgctgctcatcagcgccacctggctg
ctggg
gctgctggctgtgaaccgcgatgcactgagctttcactacctcttcgccatcttcagcggcttacagggcc
cctt
cgtcctcctttttccactgcgtgctcaaccaggaggtccggaagcacctgaagggcgtgctcggcgggag
gaagct
gcacctggaggactccgccaccaccagggccaccctgctgacgcgctccctcaactgcaacaccacc
ttcggtga
cgggcctgacatgctgcgcacagacttgggcgagtccaccgcctcgctggacagcatcgtcagggatg
aagggat

ccagaagctcggcgtgtcctctgggctggtgaggggcagccacggagagccagacgcgtccctcatg
cccaggag
ctgcaaggatccccctggccacgattccgactcagatagcgagctgtccctggatgagcagagcagct
cttacgc
ctcctcacactcgtcagacagcgaggacgatggggtgggagctgaggaaaaatggacccggccag
gggcgccgt
ccacagcacccccaaaggggacgctgtggccaaccacgttccggccggctggcccgaccagagcct
ggctgagag
tgacagtgaggaccccagcggcaagccccgcctgaaggtggagaccaaggtcagcgtggagctgc
accgcgagga
gcagggcagtcaccgtggagagtacccccggaccaggagagcgggggcgcagccaggcttgct.
agcagccagcc
cccagagcagaggaaaggcatcttgaaaaataaagtcacctacccgccgccgctgacgctgacgga
gcagacgct
gaagggccggctccgggagaagctggccgactgtgagcagagccccacatcctcgcgcacgtcttcc
ctgggctc
tggcggccccgactgcgccatcacagtcaagagccctgggagggagccggggcgtgaccacctcaa
cggggtggc
catgaatgtgcgcactgggagcgcccaggccgatggctccgactctgaaggcagtaatgaaacttcaa
tttga
>SEQID36_C-terminus
GFDLAATQDADFHEDVIHSGSALLAPATRAAWEQIQRSEGGTAQLLRRLE

GYFSNVARNVRRTYLRPFVIVTANM

ILAVDIFDKFNFTGARVPRFDTIHEEFPRELESSVSFPADFFRPPEEKEGPL

LRPAGRRTTPQTTRPGPGTEREA

PISRRRRHPDDAGQFAVALVIIYRTLGQLLPERYDPDRRSLRLPHRPIINTP

MVSTLVYSEGAPLPRPLERPVLV

EFALLEVEERTKPVCVFWNHSLAVGGTGGWSARGCELLSRNRTHVACQ

CSHTASFAVLMDISRRENGEVLPLKIV

TYAAVSLSLAALLVAFVLLSLVRMLRSNLHSIHKHLAVALFLSQLVFVIGINQ

TENPFLCTVVAILLHYIYMSTF

AWTLVESLHVYRMLTEVRNIDTGPMRFYYVVGWGIPAIVTGLAVGLDPQG

YGNPDFCWLSLQDTLIWSFAGPIGA

VIIINTVTSVLSAKVSCQRKHHYYGKKGIVSLLRTAFLLLLLISAT'WLLGLLA

VNRDALSFHYLFAIFSGLQGPF

VLLFHCVLNQEVRKHLKGVLGGRKLHLEDSATTRATLLTRSLNCNTTFGD

GPDMLRTDLGESTASLDSIVRDEGI

QKLGVSSGLVRGSHGEPDASLMPRSCKDPPGHDSDSDSELSLDEQSSS

YASSHSSDSEDDGVGAEEKWDPARGAV

HSTPKGDAVANHVPAGWPDQSLARSDSEDPSGKPRLKVETKVSVELHRE

EQGSHRGEYPPDQESGGAARLASSQP

PEQRKGILKNKVTYPPPLTLTEQTLKGRLREKLADCEQSPTSSRTSSLGS

GGPDCAITVKSPGREPGRDHLNGVA MNVRTGSAQADGSDSEGSNETSI

>SEQID37

GSNETSI

>SeqID38

AAGCACCTGAAGGgcgtgctcggcgggaggaagctgcacctggaggactccgccaccacca

gggccaccctgctgacgcgctccctcaactgcaacaccaccttcggtgacgggcctgacatgctgcgc

acagacttgggcgagtccaccgcctcgctggacagcatcgtcagggatgaagggatccagaagctcg

gcgtgtcctctgggctggtgaggggcagccacggagagccagacgcgtccctcatgcccaggagctg

caaggatccccctggccacgattccgactcagatagcgagctgtccctggatgagcagagcagctctta

cgcctcctcacactcgtcagacagcgaggacgatggggtgggagctgaggaaaaatgggacccggc

caggggcgccgtccacagcacccccaaaggggacgctgtggccaaccacgttccggccggctggcc

cgaccagagcctggctgagagtgacagtgaggaccccagcggcaagccccgcctgaaggtggaga

ccaaggtcagcgtggagctgcaccgcgaggagcagggcagtcaccgtggagagtacccccccggac

caggagagcgggggcgcagccaggcttgctagcagccagcccccagagcagaggaaaggcatctt

gaaaaataaagtcacctacccgccgccgctgacgctgacggagcagacgctgaagggccggctccg

ggagaagctggccgactgtgagcagagccccacatcctcgcgcacgtcttccctgggctctggcggcc

ccgactgcgccatcacagtcaagagccctgggagggagccggggcgtgaccacctcaacggggtg

gccatgaatgtgcgcactgggagcgcccaggccgatggctccgactctgaaggcagtaatgaaacttc

aatt

>SeqID38

khlkgvlggrklhledsattratlltrslncnttfgdgpdmlrtdlgestasldsivrdegiqklgvssglvrgshg

epdaslmprsckdppghdsdsdselsldeqsssyasshssdseddgvgaeekwdpargavhstp

kgdavanhvpagwpdqslaesdsedpsgkprlkvetkvsvelhreeqgshrgeyppdqesggaarl

assqppeqrkgilknkvtypppltlteqtlkgrlrekladceqsptssrtsslgsggpdcaitvkspgrepgr

dhlngvamnvrtgsaqadgsdsegsnetsi

## Patentansprüche

1. Verwendung einer für ein Flamingo Peptid oder Protein codierenden Nukleinsäure und/oder eines Flamingo Peptids oder Proteins zur Detektion von Krebs, insbesondere Brust-, Ovar-, und/oder Uteruskarzinomen, oder zur Detektion eines Risikos der Erkrankung an einem solchen Tumor, wobei eine Gewebeprobe, insbesondere eine Brust-, Ovar- und/oder Uterus-Gewebeprobe, auf Transkription oder Übertranskription von Flamingo RNA oder auf Expression oder Überexpression eines Flamingo Proteins untersucht wird.

2. Verwendung nach Anspruch 1, wobei eine an für Flamingo codierende Nukleinsäure oder eine an Flamingo Protein oder Peptid bindende Detektorsubstanz, vorzugsweise ent- haltend eine Reportergruppe, verwendet wird, wobei Bindung besagter Nukleinsäure und/oder besagten Proteins oder Peptids an die Detektorsubstanz halbquantitativ oder quantitativ detektiert wird.

3. Verwendung einer Flamingo RNA oder eines Flamingo Proteins oder Peptids zum Screenen nach daran bindenden Substanzen, insbesondere nach prospektiven Wirkstoffen zur Inhibierung von besagter RNA oder besagtem Protein oder Peptid oder nach prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

4. Verwendung einer Flamingo inhibierenden oder daran bindenden Substanz zur Herstellung einer pharmazeutischen Zusammensetzung zur Behandlung von Krebs, insbesondere von Brust-, Ovar-, Und/oder Uteruskarzinomen, oder zur Herstellung einer pharmazeutischen Zusammensetzung zur Diagnose eines solchen Tumors oder zur Diagnose eines Progressionsrisikos eines solchen Tumors.

5. Verwendung nach Anspruch 4, wobei die Substanz ein Antikörper ist, welcher beispielsweise durch Immunisierung eines nicht-menschlichen Säugetiers mit einem Flamingo Peptid oder Protein, mit Flamingo transfizierten Zellen, oder einer hierfür für codierenden cDNA, erhältlich ist, oder ein Phage-Display Antikörper ist.

6. Verwendung nach Anspruch 4, wobei die Substanz eine Mimikriverbindung eines Antikörpers gegen ein Flamingo Peptid oder Protein ist.

7. Verwendung nach Anspruch 4, wobei die Substanz, ein Aptamer, eine antisense RNA, eine siRNA, oder ein Ribozym ist.

8. Verwendung nach einem der Ansprüche 4 bis 7, wobei die Substanz zusätzlich eine zytotoxische und/oder immunstimulierende Komponente trägt.

9. Verwendung nach einem der Ansprüche 4 bis 8, wobei die pharmazeutische Zusammensetzung zur lokalen Applikation in Tumorzellen enthaltendem Gewebe hergerichtet ist.

10. Verfahren zur Diagnose einer Krebserkrankung, insbesondere eines Brust-, Ovar- und/oder Uteruskarzinoms, oder des Risikos der Erkrankung an einem solchen Tumor, wobei eine an Flamingo bindende Detektorsubstanz in einer Ausführungsform mit einer Reportergruppe in zu untersuchendes Gewebe appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend oder als erkrankungsgefährdet qualifiziert wird.

11. Verfahren zur Behandlung von Krebs, insbesondere eines Brust-, Uterusund/oder Ovarkarzinoms, wobei eine pharmazeutische Zusammensetzung nach einem der Ansprüche 4 bis 9 in einer physiologisch wirksamen Dosis und galenisch für die anzuwendende Darreichungsform hergerichtet einem Patienten dargereicht wird.

12. Protein oder Peptid enthaltend oder bestehend aus einer Sequenz Seq.-ID 37 oder Nukleinsäure enthaltend oder bestehend aus einer Nukleotidsequenz codierend für ein solches Peptid.
